(19) Europäisches Patentamt
European Patent Office
Office européen des brevets

(11) **EP 3 433 347 B1**

(12) **EUROPEAN PATENT SPECIFICATION**

(45) Date of publication and mention of the grant of the patent:
**06.05.2020 Bulletin 2020/19**

(21) Application number: **17713285.9**

(22) Date of filing: **23.03.2017**

(51) Int Cl.:
***C11D 3/00*** *(2006.01)* ***C11D 3/386*** *(2006.01)*
***D06M 16/00*** *(2006.01)*

(86) International application number:
**PCT/EP2017/057012**

(87) International publication number:
**WO 2017/162836 (28.09.2017 Gazette 2017/39)**

# (54) USE OF POLYPEPTIDE HAVING DNASE ACTIVITY FOR TREATING FABRICS

VERWENDUNG EINES POLYPEPTIDS MIT DNASE-AKTIVITÄT ZUR BEHANDLUNG VON GEWEBEN

UTILISATION D'UN POLYPEPTIDE AYANT UNE ACTIVITÉ DNASE POUR LE TRAITEMENT DE TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2016 DK 201600179**

(43) Date of publication of application:
**30.01.2019 Bulletin 2019/05**

(60) Divisional application:
**20163044.9**

(73) Proprietor: **Novozymes A/S**
**2880 Bagsvaerd (DK)**

(72) Inventors:
- **GORI, Klaus**
  **2880 Bagsvaerd (DK)**
- **BAUNSGAARD, Lone**
  **2880 Bagsvaerd (DK)**
- **THUESEN, Kenneth, Efterstigaard**
  **2880 Bagsvaerd (DK)**

(74) Representative: **NZ EPO Representatives**
**Krogshoejvej 36**
**2880 Bagsvaerd (DK)**

(56) References cited:
**WO-A1-2014/087011**
**WO-A1-2015/155350**
**WO-A1-2015/181286**
**WO-A1-2015/181287**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 433 347 B1

## Description

### Reference to a Sequence Listing

This application contains a Sequence Listing in computer readable form.

### Field of the Invention

The present invention concerns the use of a deoxyribonuclease (DNase), for treating a fabric item.

### Background of invention

Cleaning processes such as laundry of fabrics involve application of detergents and some degree of mechanical stress to the fabric being cleaned. Fabrics such as textile items are prone to deformations in the fabric before, during and after laundering. These deformations are non-permanent and can be removed by ironing the fabric however the fabric must be ironed upon each occasion of laundering. The deformations are commonly termed creases or wrinkles. Prior publications describe various approaches to remove creases or wrinkles from the fabric. WO2009021989 A1 (Momentive Performance Mat Inc) describes the use of polymers, such as polyurea-polysiloxane compounds for treatment of natural and synthetic fibres in a washing process to improve softness and reduce tendency to crease and WO2011005963 (Colgate Palmolive) discloses an anti-wrinkle conditioner and a method for reducing wrinkles from a heavy fabric garment while laundering. WO2006097227 (Unilever) describes fabric care compositions for ironing which reduce the wrinkling of fabrics.

However, to achieve iron-eased, soft fabrics straight from the laundry machine there is a need in the art for methods to improve the properties of fabrics.

### Summary of the Invention

The present invention relates to the use of a DNase to treat a fabric to provide improved softness and/or anti-crease properties.

### Definitions

As used herein, the singular forms "a", "an", and "the" are intended to include the plural forms as well, unless the context clearly indicates otherwise.

Anti-wrinkle and Anti-crease: In the context of the present invention, the terms "crease" and "wrinkle" and related terms, such as "anti-crease" and "anti-wrinkle", refer to non-permanent deformations in fabrics, such as textiles which can be removed by flattening at elevated temperature and moisture (e.g. by ironing). The terms are used interchangeably herein.

Bacterial: In the context of the present invention, the term "bacterial" in relation to polypeptide (such as an enzyme, e.g. a DNase) refers to a polypeptide encoded by and thus directly derivable from the genome of a bacteria, where such bacteria has not been genetically modified to encode said polypeptide, e.g. by introducing the encoding sequence in the genome by recombinant DNA technology. In the context of the present invention, the term "bacterial DNase" or "polypeptide having DNase activity obtained from a bacterial source" or "polypeptide is of bacterial origin" thus refers to a DNase encoded by and thus directly derivable from the genome of a bacterial species, where the bacterial species has not been subjected to a genetic modification introducing recombinant DNA encoding said DNase. Thus, the nucleotide sequence encoding the bacterial polypeptide having DNase activity is a sequence naturally in the genetic background of a bacterial species. A sequence encoding a bacterial polypeptide having DNase activity may also be referred to a wildtype DNase (or parent DNase). Bacterial polypeptide having DNase activity includes recombinant produced wild types. In a further aspect, the invention provides polypeptides having DNase activity, wherein said polypeptides are substantially homologous to a bacterial DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected bacterial DNase.

Biofilm: A biofilm is any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration

generally composed of extracellular DNA, proteins, and polysaccharides. Biofilms may form on living or non-living surfaces. The microbial cells growing in a biofilm are physiologically distinct from planktonic cells of the same organism, which, by contrast, are single-cells that may float or swim in a liquid medium.

Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of the community.

On laundry biofilm producing bacteria can be found among the following species: *Acinetobacter sp., Aeromicrobium sp., Brevundimonas sp., Microbacterium sp., Micrococcus luteus, Pseudomonas sp., Staphylococcus epidermidis, and Stenotrophomonas sp.* On hard surfaces biofilm producing bacteria can be found among the following species: *Acinetobacter* sp., *Aeromicrobium* sp., *Brevundimonas* sp., *Microbacterium* sp., *Micrococcus luteus, Pseudomonas* sp., *Staphylococcus epidermidis,* and *Stenotrophomonas* sp In one embodiment the biofilm producing strain is *Brevundimonas* sp.. In one embodiment the biofilm producing strain is *Pseudomonas alcaliphila* or *Pseudomonas fluorescens.*

Cellulosic textile material: The term "cellulosic textile material" means any cellulosic textile material including yarns, yarn intermediates, fibers, threads, non-woven materials, natural materials, synthetic materials, and any other textile material, fabrics made of these materials and products made from the fabrics (e.g., garments and other articles). The textile material may be in the form of knits, wovens, denims, non-wovens, felts, yarns, and towelling. The textile material is cellulose-based such as natural cellulosic material, including cotton, flax/linen, jute, ramie, sisal or coir or man-made cellulosic material (e.g., originating from wood pulp) including viscose/rayon, ramie, cellulose acetate fibers (tricell), lyocell or blends thereof. The cellulosic textile material may also be blends of cellulose based and non-cellulose based fibers, wherein the non-cellulose based material is natural polyamides including wool, camel, cashmere, mohair, rabbit and silk or a synthetic polymer such as nylon, aramid, polyester, acrylic, polypropylene and spandex elastane, or a blend thereof. Examples of blends are blends of cotton and/or rayon/viscose with one or more companion materials such as wool, synthetic fiber (e.g., polyamide fiber, acrylic fiber, polyester fiber, polyvinyl alcohol fiber, polyvinyl chloride fiber, polyurethane fiber, polyurea fiber, aramid fiber), and/or cellulose-containing fiber (e.g., rayon/viscose, ramie, flax/linen, jute, cellulose acetate fiber, lyocell).

Detergent components: the term "detergent components" is defined herein to mean the types of chemicals which can be used in detergent compositions. Examples of detergent components are alkalis, surfactants, hydrotropes, builders, co-builders, chelators or chelating agents, bleaching system or bleach components, polymers, fabric hueing agents, fabric conditioners, foam boosters, suds suppressors, dispersants, dye transfer inhibitors, fluorescent whitening agents, perfume, optical brighteners, bactericides, fungicides, soil suspending agents, soil release polymers, anti-redeposition agents, enzyme inhibitors or stabilizers, enzyme activators, antioxidants and solubilizers.

Detergent Composition: the term "detergent composition" refers to compositions that find use in the removal of undesired compounds from items to be cleaned, such as textiles. The detergent composition may be used to e.g. clean textiles for both household cleaning and industrial cleaning. The terms encompass any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; fabric fresheners; fabric softeners; and textile and laundry pre-spotters/pretreatment). In addition to containing the enzyme of the invention, the detergent formulation may contain one or more additional enzymes (such as proteases, amylases, lipases, cutinases, cellulases, endoglucanases, xyloglucanases, pectinases, pectin lyases, xanthanases, peroxidases, haloperoxygenases, catalases and mannanases, or any mixture thereof), and/or detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

DNase (deoxyribonuclease): The term "DNase" means a polypeptide with DNase activity that catalyses the hydrolytic cleavage of phosphodiester linkages in the DNA backbone, thus degrading DNA. DNase activity may be determined according to the procedure described in the Assay I. In one aspect, the DNases of the present invention have at least 20%, e.g., at least 40%, at least 50%, at least 60%, at least 70%, at least 80%, at least 90%, at least 95%, or at least 100% of the DNase activity of the polypeptide with SEQ ID NO: 1 or SEQ ID NO: 4. In one aspect of the present invention, the DNase activity is at least 105%, e.g., at least 110%, at least 120%, at least 130%, at least 140%, at least 160%, at least 170%, at least 180%, or at least 200% compared to the DNase activity of the polypeptide comprising SEQ ID NO: 1 or DNase activity of the polypeptide comprising SEQ ID NO: 4.

Fabric: The term "fabric" includes any textile material such as yarns, yarn intermediates, fibers, non-woven materials, natural materials, synthetic materials, and any other textile material, fabrics made of these materials and products made from fabrics (e.g., garments and other articles). The textile or fabric may be in the form of knits, wovens,

denims, non-wovens, felts, yarns, and towelling. The textile may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). The fabric or textile can also be made of microfiber, which is synthetic fiber made from polyesters, polyamides (e.g., nylon, Kevlar, Nomex, trogamide), or a conjugation of polyester, polyamide, and polypropylene (Prolen). Fabric may be conventional washable laundry, for example stained household laundry. When the term fabric or garment is used it is intended to include textiles as well.

Fabric improvement: The term "fabric improvement" or "textile improvement" means a benefit not directly related to catalytic stain removal or prevention of re-deposition of soils. Examples of such benefits are anti-backstaining, anti-pilling, anti-shrinkage, anti-wear, anti-wrinkle, improved color appearance, fabric softness, improved shape retention, flame or chemical resistance, anti-odor, anti-UV, water-repellency, anti-microbial, improved association between non-cellulosic and cellulosic textiles, improved static control, improved hand or texture, resistance to chemical, biological, radiological or physical hazard, and/or improved tensile strength. Prevention or reduction of dye transfer from one textile to another textile or another part of the same textile is termed anti-backstaining (also termed dye transfer inhibition). Removal of protruding or broken fibers from a textile surface to decrease pilling tendencies or remove already existing pills or fuzz is termed anti-pilling. Coating or reincorporation or smoothing of protruding or broken fibers is also termed anti-pilling. Prevention of or reduction of a decrease in dimensional size is termed anti-shrinkage. Prevention of or repair of abrasion is termed anti-wear. Prevention of wrinkles, recovery of textile from wrinkling, smoothness of seams, and/or retention of creases after repeated home laundering is termed "anti-wrinkle" or anti-crease. Improvement of the textile-softness or reduction of textile stiffness is termed improved fabric softness. Color clarification of a textile, or enhanced colorfastness to laundering, perspiration, light, chlorine and non-chlorine bleach, heat, or light at high temperature is termed improved color appearance. Resistance to dimensional size change or dimensional size change during home laundering is termed improved shape retention. Elevated combustion temperature or resistance to burning or melting at high temperatures is termed flame resistance. Resistance to chemical reactions, solubilization or degradation in the presence of chemical solvents, acid or alkali is termed chemical resistance. Resistance to adsorption or prevention of the retention of odorous compounds, particularly short chain fatty acids or low vapor pressure organic compounds is termed anti-odor. Opacity to and prevention or repair of oxidative damage caused by UV irradiation is termed anti-UV. Decreased retention of water, or resistance to wetting is termed water repellency. Enhanced microbiostatic or microbiocidal properties are termed antimicrobial. An increase in resistance to induced electrostatic charge of a textile, or increase in decay rate of an induced electrostatic charge in a textile is termed improved static control. Resistance to elongation under force or augmentation of breaking force is termed improved tensile strength.

Fungal: In the context of the present invention the term "fungal" in relation to polypeptide (such as an enzyme, e.g. a DNase) refers to a polypeptide encoded by and thus directly derivable from the genome of a fungus, where such fungus has not been genetically modified to encode said polypeptide, e.g. by introducing the encoding sequence in the genome by recombinant DNA technology. In the context of the present invention, the term "fungal DNase" or "polypeptide having DNase activity obtained from a fungal source" or "polypeptide is of fungal origin" thus refers to a DNase encoded by and thus directly derivable from the genome of a fungal species, where the fungal species has not been subjected to a genetic modification introducing recombinant DNA encoding said DNase. Thus, the nucleotide sequence encoding the fungal polypeptide having DNase activity is a sequence naturally in the genetic background of a fungal species. A sequence encoding a fungal polypeptide having DNase activity may also be referred to a wildtype DNase (or parent DNase). A fungal polypeptide having DNase activity includes recombinant produced wild types. In a further aspect, the invention provides provides polypeptides having DNase activity, wherein said polypeptides are substantially homologous to a fungal DNase. In the context of the present invention, the term "substantially homologous" denotes a polypeptide having DNase activity which is at least 80%, preferably at least 85%, more preferably at least 90%, more preferably at least 95%, even more preferably at least 96%, 97%, 98%, and most preferably at least 99% identical to the amino acid sequence of a selected fungal DNase.

Laundering: The term "laundering" relates to both household laundering and industrial laundering and means the process of treating textiles with a solution containing a cleaning or detergent composition of the present invention. The laundering process can for example be carried out using e.g. a household or an industrial washing machine or can be carried out by hand.

By the term "malodor" is meant an odor which is not desired on clean items. The cleaned item should smell fresh

and clean without malodors adhering to the item. One example of malodor is compounds with an unpleasant smell, which smell may be produced by microorganisms. Another example is unpleasant smells can be sweat or body odor adhered to an item which has been in contact with human or animal. Another example of malodor can be the odor from spices, herbs or perfumes which sticks to items for example curry or other exotic spices which smells strongly.

Mature polypeptide: The term "mature polypeptide" means a polypeptide in its final form following translation and any post-translational modifications, such as N-terminal processing, C-terminal truncation, glycosylation, phosphorylation, etc.

Mature polypeptide coding sequence: The term "mature polypeptide coding sequence" means a polynucleotide that encodes a mature polypeptide having DNase activity.

Sequence identity: The relatedness between two amino acid sequences or between two nucleotide sequences is described by the parameter "sequence identity". For purposes of the present invention, the sequence identity between two amino acid sequences may be determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), pref-erably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences may be determined using the Needleman-Wunsch algorithm (Needleman and Wunsch, 1970, supra) as implemented in the Needle program of the EMBOSS package (EM-BOSS: The European Molecular Biology Open Software Suite, Rice et al., 2000, supra), prefer-ably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment).

Wash cycle: The term "wash cycle" is defined herein as a washing operation wherein textiles are immersed in the wash liquor, mechanical action of some kind is applied to the textile in order to release stains and to facilitate flow of wash liquor in and out of the textile and finally the superfluous wash liquor is removed. After one or more wash cycles, the textile is generally rinsed and dried.

Wash liquor: The term "wash liquor" is intended to mean the solution or mixture of water and detergents optionally including enzymes used for laundrering textiles, for hard surface cleaning or for dishwashing.

**Detailed Description of the Invention**

The invention relates to the use of DNase for improving properties of fabrics such as cellulosic and/or non-cellulosic textile material. Non-limiting examples of such properties include anti-pilling, anti-shrinkage, anti-wear, color appearance, fabric softness, shape retention, static control, odor control or anti-odor and/or anti-microbial properties. WO2011/098579, WO2014/087011 and WO 2015/155350 relates to bacterial and fungal deoxyribonuclease compounds and methods for biofilm disruption and/or odor control in cleaning processes such as laundry. In particular such improved property is anti-crease and/or softness. The inventors have surprisingly found that DNases provides anti-crease and softness benefits when applied to various fabrics such as clothes and towels. Cellulosic containing materials such as cotton are particular prone to creases during laundry and/or drying and a lot of effort and time need to be used for flattening the fabric. The invention relates to the use of a DNase to treat a fabric to provide improved softness and/or anti-crease properties. Also described is a method for reducing creases that may be formed when fabrics are washed and dried. The method includes the step of contacting the fabric with a composition, such as a detergent composition, that comprises at least one DNase. The method is suitable for reducing creases of fabrics such as clothes. In particular the method is suitable for reducing creases of man-made textiles such as towels, socks, T-shirts, shirts, skirts etc. The methods is preferably a laundry method comprising the steps of:

a) adding a composition or an additive comprising DNase,
b) optionally rinsing the item.

The laundering method includes machine wash and manual wash. The laundering process is preferably carried out at temperatures from 5°C to 90°C, preferably from 15°C to 60°C, such as from 20°C to 60°C, such as from 20°C to 40°C, such as from 30°C to 60°C, such as from 30°C to 40°C, such as from 50°C to 60°C. The DNase is preferably added in an amount corresponding to range of 0.00004-100 ppm enzyme protein, such as in the range of 0.00008-100, in the range of 0.0001-100, in the range of 0.0002-100, in the range of 0.0004-100, in the range of 0.0008-100, in the range of 0.001-100 ppm enzyme protein, in the range of 0.01-100 ppm enzyme protein, in the range of 0.05-50 ppm enzyme protein, in the range of 0.1-50 ppm enzyme protein, in the range of 0.1-30 ppm enzyme protein, in the range of 0.5-20 ppm enzyme protein, in the range of 0.5-10 ppm, in the range of 0.001 ppm to 10.00 ppm, such as from 0.002 ppm to 1.00 ppm, such as from 0.002 ppm to 0.10 ppm, such as from 0.002 ppm to 0.05 ppm, such as from 0.02 ppm to 0.1 ppm, such as from 0.05 ppm to 0.1 ppm, such as from 0.1 ppm to 0.05 ppm enzyme protein. The softness of the fabric is reduced gradually after several washes. The inventors have found that polypeptides having deoxyribonuclease (DNase) activity provides anti-crease and softness benefits when applied to various fabrics. Also described is a method suitable for reducing creases and/or improving softness of fabrics such as clothes. Also described is a method for treating a fabric under industrial and institutional fabric care conditions to impart anti-crease and/or softness effect. The method is suitable for laundering various fabric items. In one particular embodiment the item is a cellulosic item such as cotton. Cotton materials are particular prone to creases during a washing process such as laundering and drying. In cellulosic fabric hydrogen bonds between the cellulose fibres may to some extend ensure that the fabric does not creases. However, hydrogen bonds are weak and can easily be broken down/re-arranged during wash resulting in laundry which is creased. However, also non-cellulosic textiles get creased during washing and drying.

Also described is an anti-crease composition comprising a DNase. The composition is preferably a detergent composition or an additive comprising a DNase. In one embodiment the composition reduces crease formation in laundered fabrics by at least 10 percent, at least 20 percent, at least 30 percent, at least 40 percent, at least 50 percent, at least 60 percent, at least 70 percent, more preferably at least 80 percent, even more preferably at least 90 percent, compared to the fabric laundered without a composition comprising DNase. The anti-crease effect can be measured as described in example 1 of the application. In one embodiment the anti-crease effect ratio of test panelists preferring fabrics washed with DNase vs test panelists preferring fabrics washed without DNase is at least 60:40, preferably at least 65:35 preferably at least 70:30 preferably at least 75:25 preferably at least 80:20 preferably at least 85:15 preferably at least 90:10 or preferably at least 95:5, when measured as described in example 1.

In one embodiment the improved softness effect ratio of test panelists preferring fabrics washed with DNase vs test panelists preferring fabrics washed without DNase is at least 60:40, preferably at least 65:35 preferably at least 70:30 preferably at least 75:25 preferably at least 80:20 preferably at least 85:15 preferably at least 90:10 or preferably at least 95:5, when measured as described in example 1.

The use of DNase can impart to the item a reduced tendency to get creased subsequent to e.g. washing or drying.

A DNase suitable for use according to the invention is preferably a microbial DNase e.g. such as a Bacillus or fungal DNase. Preferably the bacillus DNase is selected from the group consisting of polypeptides comprising the amino acid sequences shown in SEQ ID NO: 2, 3, 5, 6, 7, 8 and 9 or polypeptides having at least 60% identity, such as at least 65%, at least 70%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, or 100% sequence identity hereto.

Preferably the fungal DNase is a polypeptide comprising the amino acid sequences shown in SEQ ID NO: 1 or a polypeptide having at least 60% identity, such as at least 65%, at least 70%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, or 100% sequence identity hereto.

A suitable DNase may also be the Serratia marcescens DNase described in WO 201198579 and shown in SEQ ID NO: 4 or a polypeptide having at least 60% identity, such as at least 65%, at least 70%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99%, or 100% sequence identity hereto.

The DNase of the present invention may be present in a detergent composition in an amount corresponding to at least 0.002 mg of DNase protein, such as at least 0.004 mg of DNase protein, at least 0.006 mg of DNase protein, at least 0.008 mg of DNase protein, at least 0.01 mg of DNase protein, at least 0.1 mg of protein, preferably at least 1 mg of protein, more preferably at least 10 mg of protein, even more preferably at least 15 mg of protein, most preferably at least 20 mg of protein, and even most preferably at least 25 mg of protein. Thus, the detergent composition may comprise at least 0.00008% DNase protein, preferably at least 0.002%, 0.003%, 0.004%, 0.005%, 0.006%, 0.008%, 0.01%, 0.02%, 0.03%, 0.05%, 0.1%, 0.2%, 0.3%, 0.4%, 0.6%, 0.7%, 0.8%, 0.9% or 1.0% of DNase protein.

A DNase suitable in the present invention may be obtained from *Aspergillus,* for example from *Aspergillus oryzae.* A DNase suitable in the present invention may also be obtained from *Bacillus,* for example from *Bacillus licheniformis, Bacillus subtilis, Bacillus horikoshii, Bacillus idriensis, Bacillus cibi* and *Bacillus sp.*

In an embodiment, the present invention relates to a DNase obtained from *Aspergillus* in particular *Aspergillus oryzae.* In an embodiment, the present invention relates to a DNase polypeptide comprising the amino acid sequence of SEQ ID NO: 1 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO 1. In one aspect, the polypeptides differ by up to 10 amino acids, e.g., 1, 2, 3, 4, 5, 6, 7, 8, 9, or 10, from the polypeptide comprising SEQ ID NO: 1.

In an embodiment, the present invention relates to a DNase obtained from *Bacillus* in particular *Bacillus licheniformis.* In an embodiment, the present invention relates to a DNase polypeptide comprising the amino acid sequence of SEQ ID NO: 2 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 2. In one aspect, the polypeptides differ by up to 10 amino acids, e.g., 1, 2, 3, 4, 5, 6, 7, 8, 9, or 10, from the polypeptide comprising SEQ ID NO: 2.

In an embodiment, the present invention relates to a DNase obtained from *Bacillus* in particular *Bacillus subtilis.* In an embodiment, the present invention relates to a DNase polypeptide comprising the amino acid sequence of SEQ ID NO: 3 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 3. In one aspect, the polypeptides differ by up to 10 amino acids, e.g., 1, 2, 3, 4, 5, 6, 7, 8, 9, or 10, from the polypeptide comprising SEQ ID NO: 3.

In an embodiment, the present invention relates to a DNase obtained from *Serratia* in particular *Serratia marcescens.* In an embodiment, the present invention relates to a DNase polypeptide comprising the amino acid sequence of SEQ ID NO: 4 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 4. In one aspect, the polypeptides differ by up to 10 amino acids, e.g., 1, 2, 3, 4, 5, 6, 7, 8, 9, or 10, from the polypeptide comprising SEQ ID NO: 4.

In an embodiment, the present invention relates to a DNase obtained from *Bacillus* in particular *Bacillus idriensis.* In an embodiment, the present invention relates to a DNase polypeptide comprising the amino acid sequence of SEQ ID NO: 5 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO 5. In one aspect, the polypeptides differ by up to 10 amino acids, e.g., 1, 2, 3, 4, 5, 6, 7, 8, 9, or 10, from the polypeptide comprising SEQ ID NO: 5.

In an embodiment, the present invention relates to a DNase obtained from *Bacillus* in particular *Bacillus cibi.* In an embodiment, the present invention relates to a DNase polypeptide comprising the amino acid sequence of SEQ ID NO: 6 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 6. In one aspect, the polypeptides differ by up to 10 amino acids, e.g., 1, 2, 3, 4, 5, 6, 7, 8, 9, or 10, from the polypeptide comprising SEQ ID NO: 6.

In an embodiment, the present invention relates to a DNase obtained from *Bacillus* in particular *Bacillus horikoshii.* In an embodiment, the present invention relates to a DNase polypeptide comprising the amino acid sequence of SEQ ID NO: 7 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO 7. In one aspect, the polypeptides differ by up to 10 amino acids, e.g., 1, 2, 3, 4, 5, 6, 7, 8, 9, or 10, from the polypeptide comprising SEQ ID NO: 7.

In an embodiment, the present invention relates to a DNase obtained from *Bacillus sp.* In an embodiment, the present invention relates to a DNase polypeptide comprising the amino acid sequence of SEQ ID NO: 8 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 8. In one aspect, the polypeptides differ by up to 10 amino acids, e.g., 1, 2, 3, 4, 5, 6, 7, 8, 9, or 10, from the polypeptide comprising SEQ ID NO: 8.

In an embodiment, the present invention relates to a DNase obtained from *Bacillus sp.* In an embodiment, the present invention relates to a DNase polypeptide comprising the amino acid sequence of SEQ ID NO: 9 or comprising an amino acid sequence having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide of SEQ ID NO: 9. In one aspect, the polypeptides differ by up to 10 amino acids, e.g., 1, 2, 3, 4, 5, 6, 7, 8, 9, or 10, from the polypeptide comprising SEQ ID NO: 9.

One way of preventing, reducing or removing creases from a fabric according to the invention is by contacting the fabric with a liquid solution comprising a DNase. The fabric may be contacted with the liquid solution for example by immersing the fabric into the liquid solution. The liquid solution can be a wash liquor and the fabric may be washed at the same time. Or the liquid solution can be a softener for use when rinsing or drying a fabric. The softener may be used in the rinsing water or applied to a sheet, which is used during rinsing or drying. This embodiment of the invention is preferred for textiles such as laundry textiles which can be washed, rinsed or dried at the same time as the creases of the fabric is reduced or prevented and/or the softness is improved.

The liquid solution can also be an impregnation liquid which liquid prevents the fabric from getting creased and/or improve the softness. The liquid solution for impregnation may serve as detergent and anti-crease and/or softener solution at the same time

Non-limiting examples of compositions includes cleaning compositions such as laundry detergent composition, detergent additives, industrial cleaning compositions or a laundry softening composition.

In one embodiment, the composition is a laundry softening composition. The laundry softening composition can be applied to a textile, and may be used during washing, rinsing or drying the item.

The composition may further comprise detergent adjuvants such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed per-acids, polymeric dispersing agents, clay soil removal/anti-re-deposition agents, brighteners, suds suppressors, dyes, perfumes, structure elasticizing agents, fabric softeners, carriers, hydrotropes, builders and co-builders, fabric huing agents, anti-foaming agents, dispersants, processing aids, bacteriocides, fungicides and/or pigments.

The composition preferably comprise one or more enzymes selected from the group consisting of proteases, lipases, cutinases, amylases, carbohydrases, cellulases, pectinases, mannanases, arabinases, galactanases, xylanases, and oxidases.

Specific suitable examples of detergent ingredients and enzymes are provided below.

In one embodiment, the invention relates to a liquid detergent composition comprising at least one DNase, preferably a microbial DNase, 5 to 30 wt % surfactant, 10 to 40 wt % builder, and at least 0.01 g active detergent enzyme (such as a protease, cellulase, lipase and/or amylase) per litre of wash liquor.

The detergent enzymes may be encapsulated in a microcapsule with a semipermeable membrane, having a water activity inside these capsules (prior to addition to the liquid detergent) higher than in the liquid detergent, the capsules will undergo a (partly) collapse when added to the detergent (water is oozing out), thus leaving a more concentrated and more viscous enzyme containing interior in the capsules. The concept is very efficient in stabilizing the enzymes against hostile components in liquid detergent, and vice versa also protects enzyme sensitive components in the liquid detergent from enzymes examples of such enzymes includes, amylases, lipases, and metalloproteases. Also sensitive detergent ingredients can be encapsulated, and thus stabilized, in the microcapsules. Sensitive detergent ingredients are prone to degradation during storage. Such detergent ingredients include bleaching compounds, bleach activators, perfumes, polymers, builder, surfactants, etc.

Disclosed herein is a method for reducing or preventing creases of a fabric comprising the steps of:

a) contacting a fabric with a composition comprising at least one DNase and optionally 5 to 30 wt % surfactant, optionally 10 to 40 wt % builder, and optionally one or more detergent enzyme, such as a protease, lipase, cellulase and/or amylase.

b) optionally rinsing the item;

wherein the item is a textile, optionally a towel, tea towel kitchen cloth, cloth, oven glove, or clothes such as shirts, T-shirts, skirts etc.

The method further comprises washing the fabric with the detergent composition.

By contacting an item with a composition is meant contacting the fabric with the composition/solution for example by spraying, coating, washing, soaking or immersing the fabric with the composition according to the invention. The fabric may be contacted with the item for a short period of time such as 1-60 seconds or for a longer period of time such as 1-60 minutes or even longer such as 1-12 hours.

The pH of the liquid solution is in the range of 1 to 11, such as in the range of 5.5 to 11, such as in the range of 7 to 9, in the range of 7 to 8 or in the range of 7 to 8.5.

The temperature of the liquid solution can be in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C. In one embodiment the temperature of the liquid solution is 30°C. Common household washing temperatures is typical in the range of 20°C to 60°C, with Japan being in the lower end and Europe in the high end of the temperature range. For industrial cleaning the temperatures are often high and may be in the range of 60°C to 90°C.
In one embodiment, the fabric is rinsed after being contacted with to the composition. The fabric may be rinsed with water or with water comprising a conditioner.
The detergent composition of the present invention comprises in addition one or more DNase one or more additional cleaning composition components. The choice of additional components is within the skill of the artisan and includes conventional ingredients, including the exemplary non-limiting components set forth below.

Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and may include any conventional surfactant(s) known in the art.
When included therein, the detergent will usually contain from about 1 % to about 40% by weight of an anionic surfactant, such as from about 5% to about 30%, including from about 5% to about 15%, or from about 15% to about 20%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.
When included therein, the detergent will usually contain from about 1 % to about 40% by weigh of a cationic surfactant, for example from about 0.5% to about 30%, in particular from about 1 % to about 20%, from about 3% to about 10%, such as from about 3% to about 5%, from about 8% to about 12% or from about 10% to about 12%. Non-limiting examples of cationic surfactants include alkyldimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, ester quats, and combinations thereof.
When included therein, the detergent will usually contain from about 0.2% to about 40% by weight of a nonionic surfactant, for example from about 0.5% to about 30%, in particular from about 1 % to about 20%, from about 3% to about 10%, such as from about 3% to about 5%, from about 8% to about 12%, or from about 10% to about 12%. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof.
When included therein, the detergent will usually contain from about 0% to about 40% by weight of a semipolar surfactant. Non-limiting examples of semipolar surfactants include amine oxides (AO) such as alkyldimethylamineoxide, *N*-(coco alkyl)-*N*,*N*-dimethylamine oxide and *N*-(tallow-alkyl)-*N*,*N*-bis(2-hydroxyethyl)amine oxide, , and combinations thereof.
When included therein, the detergent will usually contain from about 0% to about 40% by weight of a zwitterionic surfactant. Non-limiting examples of zwitterionic surfactants include betaines such as alkyldimethylbetaines, sulfobetaines, and combinations thereof.

Hydrotropes

The detergent may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

Builders and Co-Builders

The detergent composition may contain about 0-65% by weight, such as about 5% to about 50% of a detergent builder or co-builder, or a mixture thereof. The builder and/or co-builder may particularly be a chelating agent that forms water-soluble complexes with Ca and Mg. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (*e.g.*, SKS-6 from Hoechst), ethanolamines such as 2-aminoethan-1-ol (MEA), diethanolamine (DEA, also known as 2,2'-iminodiethan-1-ol), triethanolamine (TEA, also known as 2,2',2"-nitrilotriethan-1-ol), and (carboxymethyl)inulin (CMI), and combinations thereof.

The detergent composition may also contain 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N*'-disuccinic acid (EDDS), methylglycinediacetic acid (MGDA), glutamic acid-*N*,*N*-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*,*N*-diacetic acid (α-ALDA), serine-*N*,*N*-diacetic acid (SEDA), isoserine-*N*,*N*-diacetic acid (ISDA), phenylalanine-*N*,*N*-diacetic acid (PHDA), anthranilic acid-*N*,*N*-diacetic acid (ANDA), sulfanilic acid-*N*,*N*-diacetic acid (SLDA), taurine-*N*,*N*-diacetic acid (TUDA) and sulfomethyl-*N*,*N*-diacetic acid (SMDA), *N*-(2-hydroxyethyl)ethylenediamine-*N*,*N*',*N*"-triacetic acid (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, e.g., WO 09/102854, US 5977053.

Bleaching systems

The detergent may contain 0-30% by weight, such as about 1 % to about 20%, of a bleaching system. Any bleaching system comprising components known in the art for use in cleaning detergents may be utilized. Suitable bleaching system components include sources of hydrogen peroxide; sources of peracids; and bleach catalysts or boosters.

Sources of hydrogen peroxide

Suitable sources of hydrogen peroxide are inorganic persalts, including alkali metal salts such as sodium percarbonate and sodium perborates (usually mono- or tetrahydrate), and hydrogen peroxide-urea (1/1).

Sources of peracids

Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.

a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone®) or alkaline earth-metal salts.

b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore acetyl triethyl citrate and triacetin have good hydrolytical stability in the product upon storage and are efficient bleach activators. Finally ATC is multifunctional, as the citrate released in the perhydrolysis reaction may function as a builder.

Bleach catalysts and boosters

The bleaching system may also include a bleach catalyst or booster.

Some non-limiting examples of bleach catalysts that may be used in the compositions include manganese oxalate, manganese acetate, manganese-collagen, cobalt-amine catalysts and manganese triazacyclononane (Mn-TACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2''-nitrilotris(ethane-1,2-diylazanylylidene-κN-methanylylidene)triphenolato-κ3O]manganese(III). The bleach catalysts may also be other metal compounds, such as iron or cobalt complexes.

In some embodiments, where a source of a peracid is included, an organic bleach catalyst or bleach booster may be used having one of the following formulae:

(i) 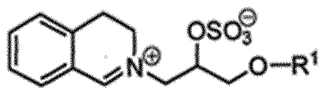

(ii) 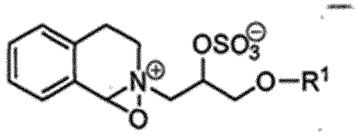

(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO2007/087258, WO2007/087244, WO2007/087259, EP1867708 (Vitamin K) and WO2007/087242. Suitable photobleaches may for example be sulfonated zinc or aluminium phthalocyanines.

Polymers

The detergent may contain 0-10% by weight, such as 0.5-5%, 2-5%, 0.5-2% or 0.2-1 % of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI).

Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

Fabric hueing agents

The detergent compositions may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO 2007/087243.

Enzymes

The detergent additive as well as the detergent composition may comprise one or more additional enzymes such as a protease, lipase, cutinase, an amylase, carbohydrase, cellulase, pectinase, mannanase, arabinase, galactanase, xylanase, oxidase, *e.g.,* a laccase, and/or peroxidase.

In general, the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

Cellulases:

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO99/001544.

Other cellulases are endo-beta-1,4-glucanase enzyme having a sequence of at least 97% identity to the amino acid sequence of position 1 to position 773 of SEQ ID NO: 2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme™, and Carezyme™ (Novozymes A/S) Carezyme Premium™ (Novozymes A/S), Celluclean ™ (Novozymes A/S), Celluclean Classic™ (Novozymes A/S), Cellusoft™ (Novozymes A/S), Whitezyme™ (Novozymes A/S), Clazinase™, and Puradax HA™ (Genencor International Inc.), and KAC-500(B)™ (Kao Corporation).

Proteases:

Suitable proteases include those of bacterial, fungal, plant, viral or animal origin e.g. vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from e.g. family M4 or other metalloprotease such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases

characterized by having a serine in the active site, which forms a covalent adduct with the substrate. The subtilases may be divided into 6 sub-divisions, i.e. the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and *subtilisin lentus, subtilisin Novo, subtilisin Carlsberg, Bacillus licheniformis, subtilisin BPN', subtilisin 309, subtilisin 147 and subtilisin 168* described in WO89/06279 and protease PD138 described in (WO93/18140). Other useful proteases may be those described in WO 92/175177, WO 01/016285, WO 02/026024 and WO 02/016547. Examples of trypsin-like proteases are trypsin (e.g. of porcine or bovine origin) and the *Fusarium* protease described in WO 89/06270, WO 94/25583 and WO 05/040372, and the chymotrypsin proteases derived from *Cellumonas* described in WO 05/052161 and WO 05/052146.

A further preferred protease is the alkaline protease from *Bacillus lentus* DSM 5483, as described for example in WO 95/23221, and variants thereof which are described in WO 92/21760, WO 95/23221, EP 1921147 and EP 1921148.

Examples of metalloproteases are the neutral metalloprotease as described in WO07/044993 (Genencor Int.) such as those derived from *Bacillus amyloliquefaciens.*

Examples of useful proteases are the variants described in: WO 92/19729, WO 96/034946, WO 98/20115, WO 98/20116, WO 99/011768, WO 01/44452, WO 03/006602, WO 04/03186, WO 04/041979, WO 07/006305, WO 11/036263, WO 11/036264, especially the variants with substitutions in one or more of the following positions: 3, 4, 9, 15, 24, 27, 42, 55, 59, 60, 66, 74, 85, 96, 97, 98, 99, 100, 101, 102, 104, 116, 118, 121, 126, 127, 128, 154, 156, 157, 158, 161, 164, 176, 179, 182, 185, 188, 189, 193, 198, 199, 200, 203, 206, 211, 212, 216, 218, 226, 229, 230, 239, 246, 255, 256, 268 and 269 wherein the positions correspond to the positions of the *Bacillus lentus* protease shown in SEQ ID NO 1 of WO 2016/001449. More preferred the subtilase variants may comprise the mutations: S3T, V4I, S9R, S9E, A15T, S24G, S24R, K27R, N42R, S55P, G59E, G59D, N60D, N60E, V66A, N74D, N85S, N85R, , G96S, G96A, S97G, S97D, S97A, S97SD, S99E, S99D, S99G, S99M, S99N, S99R, S99H, S101A, V102I, V102Y, V102N, S104A, G116V, G116R, H118D, H118N, N120S, S126L, P127Q, S128A, S154D, A156E, G157D, G157P, S158E, Y161A, R164S, Q176E, N179E, S182E, Q185N, A188P, G189E, V193M, N198D, V199I, Y203W, S206G, L211Q, L211D, N212D, N212S, M216S, A226V, K229L, Q230H, Q239R, N246K, N255W, N255D, N255E, L256E, L256D T268A, R269H. The protease variants are preferably variants of the *Bacillus lentus* protease (Savinase®) shown in SEQ ID NO 1 of WO 2016/001449, the *Bacillus amyloliquefaciens* protease (BPN') shown in SEQ ID NO 2 of WO2016/001449. The protease variants preferably have at least 80 % sequence identity to SEQ ID NO 1 or SEQ ID NO 2 of WO 2016/001449.

A protease variant comprising a substitution at one or more positions corresponding to positions 171, 173, 175, 179, or 180 of SEQ ID NO: 1 of WO2004/067737, wherein said protease variant has a sequence identity of at least 75% but less than 100% to SEQ ID NO: 1 of WO 2004/067737.

Suitable commercially available protease enzymes include those sold under the trade names Alcalase®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Blaze®, Blaze Evity® 100T, Blaze Evity® 125T, Blaze Evity® 150T, Neutrase®, Everlase® and Esperase® (Novozymes A/S), those sold under the tradename Maxatase®, Maxacal®, Maxapem®, Purafect Ox®, Purafect OxP®, Puramax®, FN2®, FN3®, FN4®, Excellase®, Excellenz P1000™ Excellenz P1250™ , Eraser®, Preferenz P100™, Purafect Prime®, Preferenz P110™, Effectenz P1000™, Purafect®™, Effectenz P1050™, Purafect Ox®™, Effectenz P2000™, Purafast®, Properase®, Opticlean® and Optimase® (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

Lipases and Cutinases:

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP 258068 and EP 305216, cutinase from *Humicola,* e.g. *H. insolens* (WO 96/13580), lipase from strains of *Pseudomonas* (some of these now renamed to *Burkholderia*), e.g. *P. alcaligenes* or *P. pseudoalcaligenes* (EP 218272), *P. cepacia* (EP 331376), *P. sp.* strain SD705 (WO 95/06720 & WO 96/27002), *P. wisconsinensis* (WO 96/12012), GDSL-type *Streptomyces* lipases (WO 10/065455), cutinase from *Magnaporthe grisea* (WO 10/107560), cutinase from *Pseudomonas mendocina* (US 5,389,536), lipase from *Thermobifida fusca* (WO 11/084412), *Geobacillus stearothermophilus* lipase (WO 11/084417), lipase from *Bacillus subtilis* (WO 11/084599), and lipase from *Streptomyces griseus* (WO 11/150157) and *S. pristinaespiralis* (WO 12/137147).

Other examples are lipase variants such as those described in EP 407225, WO 92/05249, WO 94/01541, WO 94/25578, WO 95/14783, WO 95/30744, WO 95/35381, WO 95/22615, WO 96/00292, WO 97/04079, WO 97/07202, WO 00/34450, WO 00/60063, WO 01/92502, WO 07/87508 and WO 09/109500.

Preferred commercial lipase products include include Lipolase™, Lipex™; Lipolex™ and Lipoclean™

(Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO 10/111143), acyltransferase from *Mycobacterium smegmatis* (WO 05/56782), perhydrolases from the CE 7 family (WO 09/67279), and variants of the *M. smegmatis* perhydrolase in particular the S54V variant used in the commercial product Gentle Power Bleach from Huntsman Textile Effects Pte Ltd (WO 10/100028).

Amylases:

Suitable amylases which can be used together with the DNase may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase obtained from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase obtained from *B. amyloliquefaciens* shown in SEQ ID NO: 6 of WO 2006/066594 and residues 36-483 of SEQ ID NO: 4 are those having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R,

S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme ™, Stainzyme Plus™, Natalase™, Liquozyme X and BAN™ (from Novozymes A/S), and Rapidase™, Purastar™/Effectenz™, Powerase and Preferenz S100 (from Genencor International Inc./DuPont).

<u>Peroxidases/Oxidases:</u>

A peroxidase may be a peroxidase enzyme comprised by the enzyme classification EC 1.11.1.7, as set out by the Nomenclature Committee of the International Union of Biochemistry and Molecular Biology (IUBMB), or any fragment obtained therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

A peroxidase also includes a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase may be a chloroperoxidase. Preferably, the haloperoxidase is a vanadium haloperoxidase, *i.e.,* a vanadate-containing haloperoxidase. In a preferred method the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In a preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase include, in particular, any laccase enzyme comprised by the enzyme classification EC 1.10.3.2, or any fragment obtained therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be obtained from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase obtained from *Coprinopsis* or *Myceliophthora* is preferred; in particular a laccase obtained from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising all of these enzymes. A detergent additive may be, *i.e.,* a separate additive or a combined additive, can be formulated, for example, as a granulate, liquid, slurry, etc. Preferred detergent additive formulations are granulates, in particular non-dusting granulates, liquids, in particular stabilized liquids, or slurries.

Non-dusting granulates may be produced, e.g. as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

## Microorganisms

The detergent composition may also comprise one or more microorganisms, such as one or more fungi, yeast, or bacteria.

In an embodiment, the one or more microorganisms are dehydrated (for example by lyophilization) bacteria or yeast, such as a strain of *Lactobacillus.*

In another embodiment, the microrganisms are one or more microbial spores (as opposed to vegetative cells), such as bacterial spores; or fungal spores, conidia, hypha. Preferably, the one or more spores are *Bacillus* endospores; even more preferably the one or more spores are endospores of *Bacillus subtilis, Bacillus licheniformis, Bacillus amyloliquefaciens,* or *Bacillus megaterium.*

The microrganisms may be included in the detergent composition in the same way as enzymes.

## Other materials

Any detergent components known in the art for use in detergents may also be utilized. Other optional detergent components include anti-corrosion agents, anti-shrink agents, anti-soil re-deposition agents, anti-wrinkling agents, bactericides, fungicides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in detergents may be utilized. The choice of such ingredients is well within the skill of the artisan.

## Dispersants

The detergent compositions can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

## Dye Transfer Inhibiting Agents

The detergent compositions may also include one or more dye transfer inhibiting agents. Suitable polymeric dye transfer inhibiting agents include, but are not limited to, polyvinylpyrrolidone polymers, polyamine N-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

## Fluorescent whitening agent

The detergent compositions will preferably also contain additional components that may tint articles being

cleaned, such as fluorescent whitening agent or optical brighteners. Where present the brightener is preferably at a level of about 0.01% to about 0.5%. Any fluorescent whitening agent suitable for use in a laundry detergent composition may be used in the composition. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

Soil release polymers

The detergent compositions may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279 . Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents

The detergent compositions may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Anti-static agents

The detergent compositions may also include one or more anti-static agents. Common anti-static agents are based on long-chain aliphatic amines (optionally ethoxylated) and amides, quaternary ammonium salts (e.g., behentrimonium chloride or cocamidopropyl betaine), esters of phosphoric acid, polyethylene glycol esters, or polyols. Indium tin oxide can be used as transparent anti-static coating of windows. It is also possible to use conductive polymers, like PEDOT:PSS and conducting polymer nanofibers, particularly polyaniline nanofibers.

One group of anti-static compounds are the methanesulfonamide anti-static agents substituted on the nitrogen atom and having the formula:

$$RNHSO_2CH_3$$

wherein R is a secondary aliphatic hydrocarbon chain containing at least 8 carbons.

The methanesulfonamides substituted on the nitrogen atom with one secondary long aliphatic chain containing 8-22 carbons reduces or prevents the generation of static electricity on cotton and synthetic fabrics during laundering.

These anti-static properties can be imparted to fabrics by laundering in a detergent composition containing said methanesulfonamides which are completely compatible with anionic, non-ionic, cationic and amphoteric detergents. This same treatment has been found to additionally confer a soft hand on cotton fabrics. These beneficial effects are achieved without yellowing or discoloration of the fabrics and without interference with the action of optical brighteners that may be present in the detergent composition
Another group of anti-static compounds are cationic quaternary ammonium compounds as described in WO2008/000333, WO95/29218, WO2011/011247 or WO2009/158388.

Rheology Modifiers

The detergent compositions may also include one or more rheology modifiers, structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

## Formulation of detergent products

The detergent composition may be in any convenient form, e.g., a bar, a homogenous tablet, a tablet having two or more layers, a pouch having one or more compartments, a regular or compact powder, a granule, a paste, a gel, or a regular, compact or concentrated liquid or the composition is comprised on a sheet or wipe.

Pouches can be configured as single or multi-compartments. It can be of any form, shape and material which is suitable for hold the composition, e.g. without allowing the release of the composition to release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be of blended compositions comprising hydrolytically degradable and water soluble polymer blends such as polylactide and polyvinyl alcohol (known under the Trade reference M8630 as sold by MonoSol LLC, Indiana, USA) plus plasticisers like glycerol, ethylene glycerol, propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids: US2009/0011970 A1.

Detergent ingredients can be separated physically from each other by compartments in water dissolvable pouches or in different layers of tablets. Thereby negative storage interaction between components can be avoided. Different dissolution profiles of each of the compartments can also give rise to delayed dissolution of selected components in the wash solution.

A liquid or gel detergent, which is not unit dosed, may be aqueous, typically containing at least 20% by weight and up to 95% water, such as up to about 70% water, up to about 65% water, up to about 55% water, up to about 45% water, up to about 35% water. Other types of liquids, including without limitation, alkanols, amines, diols, ethers and polyols may be included in an aqueous liquid or gel. An aqueous liquid or gel detergent may contain from 0-30% organic solvent.

A liquid or gel detergent may be non-aqueous.

## Laundry soap bars

The DNase or the composition comprising DNase may be added to laundry soap bars and used for hand washing laundry, fabrics and/or textiles. The term laundry soap bar includes laundry bars, soap bars, combo bars, syndet bars and detergent bars. The types of bar usually differ in the type of surfactant they contain, and the term laundry soap bar includes those containing soaps from fatty acids and/or synthetic soaps. The laundry soap bar has a physical form which is solid and not a liquid, gel or a powder at room temperature. The term solid is defined as a physical form which does not significantly change over time, i.e. if a solid object (e.g. laundry soap bar) is placed inside a container, the solid

object does not change to fill the container it is placed in. The bar is a solid typically in bar form but can be in other solid shapes such as round or oval.

The laundry soap bar may contain one or more additional enzymes, protease inhibitors such as peptide aldehydes (or hydrosulfite adduct or hemiacetal adduct), boric acid, borate, borax and/or phenylboronic acid derivatives such as 4-formylphenylboronic acid, one or more soaps or synthetic surfactants, polyols such as glycerine, pH controlling compounds such as fatty acids, citric acid, acetic acid and/or formic acid, and/or a salt of a monovalent cation and an organic anion wherein the monovalent cation may be for example $Na^+$, $K^+$ or $NH_4^+$ and the organic anion may be for example formate, acetate, citrate or lactate such that the salt of a monovalent cation and an organic anion may be, for example, sodium formate.

The laundry soap bar may also contain complexing agents like EDTA and HEDP, perfumes and/or different type of fillers, surfactants e.g. anionic synthetic surfactants, builders, polymeric soil release agents, detergent chelators, stabilizing agents, fillers, dyes, colorants, dye transfer inhibitors, alkoxylated polycarbonates, suds suppressers, structurants, binders, leaching agents, bleaching activators, clay soil removal agents, anti-redeposition agents, polymeric dispersing agents, brighteners, fabric softeners, perfumes and/or other compounds known in the art.

The laundry soap bar may be processed in conventional laundry soap bar making equipment such as but not limited to: mixers, plodders, e.g. a two stage vacuum plodder, extruders, cutters, logo-stampers, cooling tunnels and wrappers. The laundry soap bar is not limited to soap bars prepared by any single method. The premix may be added to the soap at different stages of the process. For example, the premix containing a soap, DNase, optionally one or more additional enzymes, a protease inhibitor, and a salt of a monovalent cation and an organic anion may be prepared and the mixture is then plodded. The DNase and optional additional enzymes may be added at the same time as the protease inhibitor for example in liquid form. Besides the mixing step and the plodding step, the process may further comprise the steps of milling, extruding, cutting, stamping, cooling and/or wrapping.

**Formulation of enzyme in co-granule**

The DNase may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulates for the detergent industry are disclosed in the IP.com disclosure IPCOM000200739D.

Another example of formulation of enzymes by the use of co-granulates are disclosed in WO 2013/188331, which relates to a detergent composition comprising (a) a multi-enzyme co-granule; (b) less than 10 wt zeolite (anhydrous basis); and (c) less than 10 wt phosphate salt (anhydrous basis), wherein said enzyme co-granule comprises from 10 to 98 wt% moisture sink component and the composition additionally comprises from 20 to 80 wt% detergent moisture sink component.

WO 2013/188331 also relates to a method of treating and/or cleaning a surface, preferably a fabric surface comprising the steps of (i) contacting said surface with the detergent composition as claimed and described herein in an aqueous wash liquor, (ii) rinsing and/or drying the surface.

The multi-enzyme co-granule may comprise a DNase and (a) one or more enzymes selected from the group consisting of first- wash lipases, cleaning cellulases, xyloglucanases, perhydrolases, peroxidases, lipoxygenases, laccases and mixtures thereof; and (b) one or more enzymes selected from the group consisting of hemicellulases, proteases, care cellulases, cellobiose dehydrogenases, xylanases, phospho lipases, esterases, cutinases, pectinases, mannanases, pectate lyases, keratinases, reductases, oxidases, phenoloxidases, ligninases, pullulanases, tannases, pentosanases, lichenases glucanases, arabinosidases, hyaluronidase, chondroitinase, amylases, and mixtures thereof.

**DETERGENT COMPOSITIONS**

The below mentioned detergent composition can be used in combination with the polypeptide of the invention for preventing or reducing creases and wrinkles in laundry.

**Composition of Ariel Sensitive White & Color, liquid detergent composition:**

Aqua, Alcohol Ethoxy Sulfate, Alcohol Ethoxylate, Amino Oxide, Citrid Acid, C12-18 topped palm kernel fatty acid, Protease, Glycosidase, Amylase, Ethanol, 1,2 Propanediol, Sodium Formate, Calcium Chloride, Sodium hydroxide, Silicone Emulsion, Trans-sulphated EHDQ (the ingredients are listed in descending order).

**Composition of WFK IEC-A model detergent (powder)**

Ingredients: Linear sodium alkyl benzene sulfonate 8,8 %, Ethoxylated fatty alcohol C12-18 (7 EO) 4,7 %,

Sodium soap 3,2 %, Anti foam DC2-4248S 3,9 %, Sodium aluminium silicate zeolite 4A 28,3 %, Sodium carbonate 11,6 %, Sodium salt of a copolymer from acrylic and maleic acid (Sokalan CP5) 2,4 %, Sodium silicate 3,0 %, Carboxymethylcellulose 1,2 %, Dequest 2066 2,8 %, Optical whitener 0,2 %, Sodium sulfate6,5 %, Protease 0,4 %.

**Composition of model detergent A (liquid)**

Ingredients: 12% LAS, 11% AEO Biosoft N25-7 (NI), 7% AEOS (SLES), 6% MPG (monopropylene glycol), 3% ethanol, 3% TEA, 2.75% cocoa soap, 2.75% soya soap, 2% glycerol, 2% sodium hydroxide, 2% sodium citrate, 1% sodium formiate, 0.2% DTMPA and 0.2% PCA (all percentages are w/w)

**Composition of Ariel Actilift (liquid)**

Ingredients: 5-15% Anionic surfactants; <5% Non-ionic surfactants, Phosphonates, Soap; Enzymes, Optical brighteners, Benzisothiazolinone, Methylisothiazolinone, Perfumes, Alpha-isomethyl ionone, Citronellol, Geraniol, Linalool.

**Composition of Ariel Actilift Colour & Style**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, Hydrogenated Castor Oil, Sodium Diethylenetriamine Pentamethylene Phosphonate, PEG/PPG-10/2 Propylheptyl Ether, Butyophenyl Methylpropional, Polyvinylpyridine-N-Oxide, Sorbitol, Glycerin, Ethanolamine, Sodium Hydroxide, Alpha-Isomethyl Ionone, Protease, Calcium Chloride, Geraniol, Linalool, Citronelllol, Tripropylene Glycol, Glycosidase, Benzisothiazolinone, Dimethicone, Glycosidase, Sodium Acetate, Cellulase, Colorant, Glyceryl Stearate, Hydroxyethylcellulose, Silica.

**Composition of Ariel Actilift Colour & Style, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Polyvinylpyridine-N-Oxide, Sorbitol, Calcium Chloride, protease, Glycerin, Glucosidase, Glycosidase, Sodium Acetate, Colorant, Cellulase.

**Composition of Ariel Actilift Whites & Colours Coolclean, new pack**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, Perfume, Sorbitol, Calcium Chloride, protease, Glycerin, Glucosidase, Glycosidase, Sodium Acetate, Colorant, Cellulase.

**Composition of Ariel Sensitive White & Color**

Ingredients: Aqua, Sodium Laureth Sulfate, Propylene Glycol, C14-C15 Pareth-7, Sodium citrate, Sodium Palm Kernelate, Alcohol, Sodium Formate, Sulfated Ethoxylated Hexamethylenediamine Quaternized, Sodium Hydroxide, , Sorbitol, Calcium Chloride, protease, Glycerin, Glycosidase, Sodium Acetate, Cellulase, Silica.

**Composition of Ariel Actilift, regular**

Aqua, Sodium Dodecylbenzenesulfonate, C14-C15 Pareth-7, Sodium Citrate, Propylene Glycol, Sodium Palm Kernelate, Sodium Laureth Sulfate, MEA Dodecylbenzenesulfonage, Sulfated Ethoxylated Hexamethylenediamine Quaternized, Sodium Cumenesulfonate, Perfume, Co-polymer of PEG/Vinyl Acetate, Sodium formate, C12-C14 Pareth-7, Hydrogenated Castor Oil, Sodium Diethylenetriamine Pentamethylene Phosphonate, PEG/PPG-10/2 Propylheptyl Ether, Butyophenyl Methylpropional, Fluorescent Brightener 9, Sorbitol, Glycerin, Ethanolamine, Sodium Hydroxide, Alpha-Isomethyl Ionone, Protease, Calcium Chloride, Geraniol, Linalool, Citronelllol, Tripropylene Glycol, Sodium Chloride, Glycosidase, Benzisothiazolinone, Dimethicone, Glycosidase, Sodium Acetate, Cellulase, Colorant, Glyceryl Stearate, Hydroxyethylcellulose, Silica.

**Composition of Persil Small & Mighty (liquid)**

Ingredients: 15-30% Anionic surfactants, Non-ionic surfacts, 5-15% Soap, < 5% Polycarboxylates, Perfume, Phosphates, Optical Brighteners

**Composition of Fairy Non Bio (liquid)**

Ingredients: 15-30% Anionic Surfactants,5-15% Non-Ionic Surfactants, Soap, Benzisothiazolinone, Methylisothiazolinone, Perfumes

**Composition of Model detergent T (powder)**

Ingredients: 11% LAS, 2% AS/AEOS, 2% soap, 3% AEO, 15.15% sodium carbonate, 3% sodium slilcate, 18.75% zeolite, 0.15% chelant, 2% sodium citrate, 1.65% AA/MA copolymer, 2.5% CMC and 0.5% SRP (all percentages are w/w).

**Composition of Model detergent X (powder)**

Ingredients: 16.5% LAS, 15% zeolite, 12% sodium disilicate, 20% sodium carbonate, 1% sokalan, 35.5% sodium sulfate (all percentages are w/w).

**Composition of Ariel Actilift (powder)**

Ingredients: 15-30% Anionic surfactants, <5% Non-ionic surfactants, Phosphonates, Polycarboxylates, Zeolites; Enzymes, Perfumes, Hexyl cinnamal.

**Composition of Persil Megaperls (powder)**

Ingredients: 15 - 30 % of the following: anionic surfactants, oxygen-based bleaching agent and zeolites, less than 5 % of the following: non-ionic surfactants, phosphonates, polycarboxylates, soap, Further ingredients: Perfumes, Hexyl cinnamal, Benzyl salicylate, Linalool, optical brighteners, Enzymes and Citronellol.

**Gain Liquid, Original:**

Ingredients: Water, Alcohol Ethoxysulfate, Diethylene Glycol, Alcohol Ethoxylate, Ethanolamine, Linear Alkyl Benzene Sulfonate, Sodium Fatty Acids, Polyethyleneimine Ethoxylate, Citric Acid, Borax, Sodium Cumene Sulfonate, Propylene Glycol, DTPA, Disodium Diaminostilbene Disulfonate, Dipropylethyl Tetramine, Sodium Hydroxide, Sodium Formate, Calcium Formate, Dimethicone, Amylase, Protease, Liquitint™ , Hydrogenated Castor Oil, Fragrance

**Tide Liquid, Original:**

Ingredients: Linear alkylbenzene sulfonate, propylene glycol, citric acid, sodium hydroxide, borax, ethanolamine, ethanol, alcohol sulfate, polyethyleneimine ethoxylate, sodium fatty acids, diquaternium ethoxysulfate, protease, diethylene glycol, laureth-9, alkyldimethylamine oxide, fragrance, amylase, disodium diaminostilbene disulfonate, DTPA, sodium formate, calcium formate, polyethylene glycol 4000, mannanase, Liquitint™ Blue, dimethicone.

**Liquid Tide, Free and Gentle:** Water, sodium alcoholethoxy sulfate, propylene glycol, borax, ethanol, linear alkylbenzene sulfonate sodium, salt, polyethyleneimine ethoxylate, diethylene glycol, trans sulfated & ethoxylated hexamethylene diamine, alcohol ethoxylate, linear alkylbenzene sulfonate, MEA salt, sodium formate, sodium alkyl sulfate, DTPA, amine oxide, calcium formate, disodium diaminostilbene, disulfonate, amylase, protease, dimethicone, benzisothiazolinone

**Tide Coldwater Liquid, Fresh Scent:** Water, alcoholethoxy sulfate, linear alkylbenzene sulfonate, diethylene glycol, propylene glycol, ethanolamine, citric acid, Borax, alcohol sulfate, sodium hydroxide, polyethyleneimine, ethoxylate, sodium fatty acids, ethanol, protease, Laureth-9, diquaternium ethoxysulfate, lauramine oxide, sodium cumene, sulfonate, fragrance, DTPA, amylase, disodium, diaminostilbene, disulfonate, sodium formate, disodium distyrylbiphenyl disulfonate, calcium formate, polyethylene glycol 4000, mannanase, pectinase, Liquitint™ Blue, dimethicone

**Tide TOTALCARE™ Liquid, Cool Cotton:** Water, alcoholethoxy sulfate, propylene glycol, sodium fatty acids, laurtrimonium chloride, ethanol, sodium hydroxide, sodium cumene sulfonate, citric acid, ethanolamine, diethylene glycol, silicone polyether, borax, fragrance, polyethyleneimine ethoxylate, protease, Laureth-9, DTPA, polyacrylamide quaternium chloride, disodium diaminostilbene disulfonate, sodium formate, Liquitint™ Orange, dipropylethyl tetraamine, dimethicone, cellulase,

**Liquid Tide Plus Bleach Alternative™, Vivid White and Bright, Original and Clean Breeze:** Water, sodium alcoholethoxy sulfate, sodium alkyl sulfate, MEA citrate, linear alkylbenzene sulfonate, MEA salt, propylene glycol, diethylene glycol, polyethyleneimine ethoxylate, ethanol, sodium fatty acids, ethanolamine, lauramine oxide, borax, Laureth-9, DTPA, sodium cumene sulfonate, sodium formate, calcium formate, linear alkylbenzene sulfonate, sodium salt, alcohol sulfate, sodium hydroxide, diquaternium ethoxysulfate, fragrance, amylase, protease, mannanase, pectinase, disodium diaminostilbene disulfonate, benzisothiazolinone, Liquitint™ Blue, dimethicone, dipropylethyl tetraamine.

**Liquid Tide HE, Original Scent:** Water, Sodium alcoholethoxy sulfate, MEA citrate, Sodium Alkyl Sulfate, alcohol ethoxylate, linear alkylbenzene sulfonate, MEA salt, sodium fatty acids, polyethyleneimine ethoxylate, diethylene glycol, propylene glycol, diquaternium ethoxysulfate, borax, polyethyleneimine, ethoxylate propoxylate, ethanol, sodium cumene sulfonate, fragrance, DTPA, disodium diaminostilbene disulfonate, Mannanase, cellulase, amylase, sodium formate, calcium formate, Lauramine oxide, Liquitint™ Blue, Dimethicone / polydimethyl silicone.

**Tide TOTALCARE HE Liquid, renewing Rain:**Water, alcoholethoxy sulfate, linear alkylbenzene sulfonate, alcohol ethoxylate, citric acid, Ethanolamine, sodium fatty acids, diethylene glycol, propylene glycol, sodium hydroxide, borax, polyethyleneimine ethoxylate, silicone polyether, ethanol, protease, sodium cumene sulfonate, diquaternium ethoxysulfate, Laureth-9, fragrance, amylase, DTPA, disodium diaminostilbene disulfonate, disodium distyrylbiphenyl disulfonate, sodium formate, calcium formate, mannanase, Liquitint™ Orange, dimethicone, polyacrylamide quaternium chloride, cellulase, dipropylethyl tetraamine.

**Tide liquid HE Free:** Water, alcoholethoxy sulfate, diethylene glycol, monoethanolamine citrate, sodium formate, propylene glycol, linear alkylbenzene sulfonates, ethanolamine, ethanol, polyethyleneimine ethoxylate, amylase, benzisothiazolin, borax, calcium formate, citric acid, diethylenetriamine pentaacetate sodium, dimethicone, diquaternium ethoxysulfate, disodium diaminostilbene disulfonate, Laureth-9, mannanase, protease, sodium cumene sulfonate, sodium fatty acids.

**Tide Coldwater HE Liquid, Fresh Scent:** Water, alcoholethoxy sulfate, MEA Citrate, alcohol sulfate, Alcohol ethoxylate, Linear alkylbenzene sulfonate MEA, sodium fatty acids, polyethyleneimine ethoxylate, diethylene glycol, propylene glycol, diquaternium ethoxysulfate, borax, polyethyleneimine ethoxylate propoxylate, ethanol, sodium cumene sulfonate, fragrance, DTPA, disodium diaminostilbene disulfonate, protease, mannanase, cellulase, amylase, sodium formate, calcium formate, lauramine oxide, Liquitint™ Blue, dimethicone.

**Tide for Coldwater HE Free Liquid:** Water, sodium alcoholethoxy sulfate, MEA Citrate, Linear alkylbenzene sulfonate: sodium salt, Alcohol ethoxylate, Linear alkylbenzene sulfonate: MEA salt, sodium fatty acids, polyethyleneimine ethoxylate, diethylene glycol, propylene glycol, diquaternium ethoxysulfate, Borax, protease, polyethyleneimine ethoxylate propoxylate, ethanol, sodium cumene sulfonate, Amylase, citric acid, DTPA, disodium diaminostilbene disulfonate, sodium formate, calcium formate, dimethicone.

**Tide Simply Clean & Fresh:** Water, alcohol ethoxylate sulfate, linear alkylbenzene sulfonate Sodium/Mea salts, propylene glycol, diethylene glycol, sodium formate, ethanol, borax, sodium fatty acids, fragrance, lauramine oxide, DTPA, Polyethylene amine ethoxylate, calcium formate, disodium diaminostilbene disulfonate, dimethicone, tetramine, Liquitint™ Blue.

**Tide Pods, Ocean Mist, Mystic Forest, Spring Meadow: L**inear alkylbenzene sulfonates, C12-16 Pareth-9, propylene glycol, alcoholethoxy sulfate, water, polyethyleneimine ethoxylate, glycerine, fatty acid salts, PEG-136 polyvinyl acetate, ethylene Diamine disuccinic salt, monoethanolamine citrate, sodium bisulfite, diethylenetriamine pentaacetate sodium, disodium distyrylbiphenyl disulfonate, calcium formate, mannanase, exyloglucanase, sodium formate, hydrogenated castor oil, natalase, dyes, termamyl, subtilisin, benzisothiazolin, perfume.

**Tide to Go:** Deionized water, Dipropylene Glycol Butyl Ether, Sodium Alkyl Sulfate, Hydrogen Peroxide, Ethanol, Magnesium Sulfate, Alkyl Dimethyl Amine Oxide, Citric Acid, Sodium Hydroxide, Trimethoxy Benzoic Acid, Fragrance.

**Tide Stain Release Liquid:** Water, Alkyl Ethoxylate, Linear Alkylbenzenesulfonate, Hydrogen Peroxide, Diquaternium Ethoxysulfate, Ethanolamine, Disodium Distyrylbiphenyl Disulfonate, tetrabutyl Ethylidinebisphenol, F&DC Yellow 3, Fragrance.

**Tide Stain Release Powder:** Sodium percarbonate, sodium sulfate, sodium carbonate, sodium aluminosilicate, nonanoyloxy benzene sulfonate, sodium polyacrylate, water, sodium alkylbenzenesulfonate, DTPA, polyethylene glycol, sodium palmitate, amylase, protease, modified starch, FD&C Blue 1, fragrance.

**Tide Stain Release, Pre Treater Spray:**

Water, Alkyl Ethoxylate, MEA Borate, Linear Alkylbenzenesulfonate, Propylene Glycol, Diquaternium Ethoxysulfate, Calcium Chlorideenzyme, Protease, Ethanolamine, Benzoisothiazolinone, Amylase, Sodium Citrate, Sodium Hydroxide, Fragrance.

**Tide to Go Stain Eraser:** Water, Alkyl Amine Oxide, Dipropylene Glycol Phenyl Ether, Hydrogen Peroxide,

Citric Acid, Ethylene Diamine Disuccinic Acid Sodium salt, Sodium Alkyl Sulfate, Fragrance.

**Tide boost with Oxi:**

Sodium bicarbonate, sodium carbonate, sodium percarbonate, alcohol ethoxylate, sodium chloride, maleic/acrylic copolymer, nonanoyloxy benzene sulfonate, sodium sulfate, colorant, diethylenetriamine pentaacetate sodium salt, hydrated aluminosilicate (zeolite), polyethylene glycol, sodium alkylbenzene sulfonate, sodium palmitate, starch, water, fragrance.

**Tide Stain Release boost Duo Pac:**

Polyvinyl Alcoholpouch film, wherein there is packed a liquid part and a powder part:

**Liquid Ingredients:** Dipropylene Glycol, diquaternium Ethoxysulfate, Water, Glycerin, LiquitintTM Orange, **Powder Ingredients:** sodium percarbonate, nonanoyloxy benzene sulfonate, sodium carbonate, sodium sulfate, sodium aluminosilicate, sodium polyacrylate, sodium alkylbenzenesulfonate, maleic/acrylic copolymer, water, amylase, polyethylene glycol, sodium palmitate, modified starch, protease, glycerine, DTPA, fragrance.

**Tide Ultra Stain Release:** Water, sodium alcoholethoxy sulfate, linear alkyl benzene sulfonate, sodium/MEA salts, MEA citrate, propylene glycol, polyethyleneimine ethoxylate, ethanol, diethylene glycol, polyethyleneimine propoxyethoxylate, sodium fatty acids, protease, borax, sodium cumene sulfonate, DTPA, fragrance, amylase, disodium diaminostilbene disulfonate, calcium formate, sodium formate, gluconase, dimethicone, Liquitint™ Blue, mannanase.

**Ultra Tide with a Touch of Downy® Powdered Detergent, April Fresh/Clean Breeze/April Essence:** Sodium Carbonate, Sodium Aluminosilicate, Sodium Sulfate, Linear Alkylbenzene Sulfonate, Bentonite, Water, Sodium Percarbonate, Sodium Polyacrylate, Silicate, Alkyl Sulfate, Nonanoyloxybenzenesulfonate, DTPA, Polyethylene Glycol 4000, Silicone, Ethoxylate, fragrance, Polyethylene Oxide, Palmitic Acid, Disodium Diaminostilbene Disulfonate, Protease, Liquitint™ Red, FD&C Blue 1, Cellulase.

**Ultra Tide with a Touch of Downy Clean Breeze:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimine, propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, sodium formate, Liquitint™ Blue.

**Ultra Tide with Downy Sun Blossom:** Water, sodium alcoholethoxy sulfate, MEA citrate, linear alkyl benzene sulfonate: sodium/MEA salts, propylene glycol, ethanol, diethylene glycol, polyethyleneimine propoxyethoxylate, polyethyleneimine ethoxylate, alcohol sulfate, dimethicone, fragrance, borax, sodium fatty acids, DTPA, protease, sodium bisulfite, disodium diaminostilbene disulfonate, amylase, castor oil, calcium formate, MEA, styrene acrylate copolymer, propanaminium propanamide, gluconase, sodium formate, Liquitint™ Blue.

**Ultra Tide with Downy April Fresh/ Sweet Dreams:** Water, sodium alcoholethoxy sulfate, MEA citrate, linear alkyl benzene sulfonate: sodium/MEA salts, propylene glycol, polyethyleneimine ethoxylate, ethanol, diethylene glycol, polyethyleneimin propoxyethoxylate, diquaternium ethoxysulfate, alcohol sulfate, dimethicone, fragrance, borax, sodium fatty acids, DTPA, protease, sodium bisulfite, disodium diaminostilbene disulfonate, amylase, gluconase, castor oil, calcium formate, MEA, styrene acrylate copolymer, propanaminium propanamide, sodium formate, Liquitint™ Blue.

**Ultra Tide Free Powdered Detergent:** Sodium Carbonate, Sodium Aluminosilicate, Alkyl Sulfate, Sodium Sulfate, Linear Alkylbenzene Sulfonate, Water, Sodium polyacrylate, Silicate, Ethoxylate, Sodium percarbonate, Polyethylene Glycol 4000, Protease, Disodium Diaminostilbene Disulfonate, Silicone, Cellulase.

**Ultra Tide Powdered Detergent, Clean Breeze/Spring Lavender/mountain Spring:** Sodium Carbonate, Sodium Aluminosilicate, Sodium Sulfate, Linear Alkylbenzene Sulfonate, Alkyl Sulfate, Sodium Percarbonate, Water, Sodium Polyacrylate, Silicate, Nonanoyloxybenzenesulfonate, Ethoxylate, Polyethylene Glycol 4000, Fragrance, DTPA, Disodium Diaminostilbene Disulfonate, Palmitic Acid, Protease, Silicone, Cellulase.

**Ultra Tide HE (high Efficiency) Pwdered Detergent, Clean Breeze:** Sodium Carbonate, Sodium Aluminosilicate,

Sodium Sulfate, Linear Alkylbenzene Sulfonate, Water, Nonanoyloxybenzenesulfonate, Alkyl Sulfate, Sodium Polyacrylate, Silicate, Sodium Percarbonate, Ethoxylate, Polyethylene Glycol 4000, Fragrance, DTPA, Palmitic Acid, Disodium Diaminostilbene Disulfonate, Protease, Silicone, Cellulase.

**Ultra Tide Coldwater Powdered Detergent, Fresh Scent:** Sodium Carbonate, Sodium Aluminosilicate, Sodium Sulfate, Sodium Percarbonate, Alkyl Sulfate, Linear Alkylbenzene Sulfonate, Water, Nonanoyloxybenzenesulfonate, Sodium Polyacrylate, Silicate, Ethoxylate, Polyethylene Glycol 4000, DTPA, Fragrance, Natalase, Palmitic Acid, Protease, Disodium, Diaminostilbene Disulfonate, FD&C Blue 1, Silicone, Cellulase, Alkyl Ether Sulfate.

**Ultra Tide with bleach Powdered Detergent, Clean Breeze:** Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate, Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

**Ultra Tide with Febreeze Freshness™ Powdered Detergent, Spring Renewal:** Sodium Carbonate, Sodium Aluminosilicate, Sodium Sulfate, Linear Alkylbenzene Sulfonate, Sodium Percarbonate , Alkyl Sulfate, Water, Sodium Polyacrylate, Silicate, Nonanoyloxybenzenesulfonate, Ethoxylate, Polyethylene Glycol 4000, DTPA, Fragrance, Cellulase, Protease, Disodium Diaminostilbene Disulfonate, Silicone, FD&C Blue 1.

**Liquid Tide Plus with Febreeze Freshness - Sport HE Active Fresh:**
Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, Ethanol, sodium cumene sulfonate, borax, fragrance, DTPA, Sodium bisulfate, disodium diaminostilbene disulfonate, Mannanase, cellulase, amylase, sodium formate, calcium formate, Lauramine oxide, Liquitint™ Blue, Dimethicone / polydimethyl silicone.

**Tide Plus Febreeze Freshness Spring & Renewal:**
Water, sodium alcoholethoxy sulfate, linear alkyl benzene sulfonate: sodium/MEA salts, MEA citrate, propylene glycol, polyethyleneimine ethoxylate, fragrance, ethanol, diethylene glycol, polyethyleneimine propoxyethoxylate, protease, alcohol sulfate, borax, sodium fatty acids, DTPA, disodium diaminostilbene disulfonate, MEA, mannanase, gluconase, sodium formate, dimethicone, Liquitint™ Blue, tetramine.

**Liquid Tide Plus with Febreeze Freshness, Sport HE Victory Fresh:** Water, Sodium alcoholethoxy sulfate, MEA citrate, linear alkylbenzene sulfonate, sodium salt, linear alkylbenzene sulfonate: MEA salt, alcohol ethoxylate, sodium fatty acids, propylene glycol, diethylene glycol, polyethyleneimine ethoxylate propoxylate, diquaternium ethoxysulfate, ethanol, sodium cumene sulfonate, borax, fragrance, DTPA, Sodium bisulfate, disodium diaminostilbene disulfonate, Mannanase, cellulase, amylase, sodium formate, calcium formate, Lauramine oxide, Liquitint™ Blue, Dimethicone / polydimethyl silicone.

**Tide Vivid White + Bright Powder, Original:**

Sodium Carbonate, Sodium Aluminosilicate, Sodium Sulfate, Linear Alkylbenzene Sulfonate, Sodium Percarbonate, Nonanoyloxybenzenesulfonate, Alkyl Sulfate, Water, Silicate, Sodium Polyacrylate

Ethoxylate, Polyethylene Glycol 4000, Fragrance, DTPA, Palmitic Acid, Protease, Disodium Diaminostilbene Disulfonate, Silicone, FD&C Blue 1, Cellulase, Alkyl Ether Sulfate.

**Hey Sport Tex Wash Detergent**
Aqua, dodecylbenzenesulfonsäure, laureth-11, peg-75 lanolin, propylene glycol, alcohol denat., potassium soyate, potassium hydroxide, disodium cocoamphodiacetate, ethylendiamine triacetate cocosalkyl acetamide, parfum, zinc ricinoleate, sodium chloride, benzisothiazolinone, methylisothiazolinone, ci 16255, benzyl alcohol.

The products named Tide, Ariel, Gain and Fairy are commercially available products supplied by Procter & Gamble. The products named Persil are commercially available products supplied by Unilever and Henkel. The products named Hey Sport are commercially available products supplied by Hey Sport.

Table 1.

| Ingredient | Amount (in wt %) |
|---|---|
| Anionic detersive surfactant (such as alkyl benzene sulphonate, alkyl ethoxylated sulphate and mixtures | from 8% to 15% |
| Non-ionic detersive surfactant (such as alkyl ethoxylated alcohol) | from 0.5% to 4% |
| Cationic detersive surfactant (such as quaternary ammonium compounds) | from 0 to 4% |
| Other detersive surfactant (such as zwiterionic detersive surfactants, amphoteric surfactants and mixtures thereof) | from 0% to 4% |
| Carboxylate polymer (such as co-polymers of maleic acid and acrylic acid) | from 1 % to 4% |
| Polyethylene glycol polymer (such as a polyethylene glycol polymer comprising poly vinyl acetate side chains) | from 0.5% to 4% |
| Polyester soil release polymer (such as Repel-o-tex from and/or Texcare polymers) | from 0.1 to 2% |
| Cellulosic polymer (such as carboxymethyl cellulose, methyl cellulose and combinations thereof) | from 0.5% to 2% |
| Other polymer (such as amine polymers, dye transfer inhibitor polymers, hexamethylenediamine derivative polymers, and mixtures thereof) | from 0% to 4% |
| Zeolite builder and phosphate builder (such as zeolite 4A and/or sodium tripolyphosphate) | from 0% to 4 wt% |
| Other builder (such as sodium citrate and/or citric acid) | from 0% to 3% |
| Carbonate salt (such as sodium carbonate and/or sodium bicarbonate) | from 15% to 30% |
| Silicate salt (such as sodium silicate) | from 0% to 10% |
| Filler (such as sodium sulphate and/or bio-fillers) | from 10% to 40% |
| Source of available oxygen (such as sodium percarbonate) | from 10% to 20% |
| Bleach activator (such as tetraacetylethylene diamine (TAED) and/or nonanoyloxybenzenesulphonate (NOBS) | from 2% to 8% |
| Bleach catalyst (such as oxaziridinium-based bleach catalyst and/or transition metal bleach catalyst) | from 0% to 0.1% |
| Other bleach (such as reducing bleach and/or pre-formed peracid) | from 0% to 10% |
| Chelant (such as ethylenediamine-N'N'-disuccinic acid (EDDS) and/or hydroxyethane diphosphonic acid(HEDP) | from 0.2% to1 % |
| Photobleach (such as zinc and/or aluminium sulphonated phthalocyanine) | from 0% to 0.1% |
| Hueing agent (such as direct violet 99, acid red 52, acid blue 80, direct violet 9, solvent violet 13 and any combination thereof) | from 0% to 1% |
| Brightener (such as brightener 15 and/or brightener 49) | from 0.1% to 0.4% |
| Protease such as those mentioned under the heading "proteases" e.g. Savinase, Savinase Ultra, Ovozyme, Kannase, Liquanase, Polarzyme, Purafect, Properase, FN3, FN4 and any combination thereof) | from 0.1% to 0.4% |
| Amylase (such as Termamyl, Termamyl ultra Natalase, Optisize, Stainzyme, Stainzyme Plus, and any combination thereof) | from 0.05% to 0.2% |
| Cellulase (such as Carezyme and/or Celluclean) | from 0.05% to 0.2% |
| Lipase (such as Lipex, Lipolex, Lipoclean and any combination thereof) | from 0.2 to 1 % |
| Other enzyme (such as xyloglucanase, cutinase, pectate lyase, mannanase, bleaching enzyme) | from 0% to 2% |
| Fabric softener (such as montmorillonite clay and/or polydimethylsiloxane (PDMS) | from 0% to 4% |
| Flocculant (such as polyethylene oxide) | from 0% to 1% |

(continued)

| Ingredient | Amount (in wt %) |
|---|---|
| Suds suppressor (such as silicone and/or fatty acid) | from 0% to 0.1 % |
| Perfume (such as perfume microcapsule, spray-on perfume, starch encapsulated perfume accords, perfume loaded zeolite, and any combination thereof) | from 0.1% to 1% |
| Aesthetics (such as coloured soap rings and/or coloured speckles/noodles) | from 0% to 1% |
| Miscellaneous | balance |

Table 2.

| Ingredient | Amount |
|---|---|
| Carboxyl group-containing polymer (comprising from about 60% to about 70% by mass of an acrylic acid-based monomer (A); and from about 30% to about 40%) by mass of a sulfonic acid group-containing monomer (B); and wherein the average molecular weight is from about 23,000 to about 50,000 preferably in the range of from about 25,000 to about 38,000 as described in WO2014032269. | from about 0.5 wt% to about 1.5 wt% |
| Amylase (Stainzyme Plus(R), having an enzyme activity of 14 mg active enzyme/ g) | from about 0.1wt% to about 0.5 wt% |
| Anionic detersive surfactant (such as alkyl benzene sulphonate, alkyl ethoxylated sulphate and mixtures thereof) | from about 8 wt% to about 15 wt% |
| Non-ionic detersive surfactant (such as alkyl ethoxylated alcohol) | from about 0.5 wt% to 4 wt% |
| Cationic detersive surfactant (such as quaternary ammonium compounds) | from about 0 wt% to about 4 wt% |
| Other detersive surfactant (such as zwiterionic detersive surfactants, amphoteric surfactants and mixtures thereof) | from about 0 wt% to 4 wt% |
| Carboxylate polymer (such as co-polymers of maleic acid and acrylic acid) | from about 1 wt% to about 4 wt% |
| Polyethylene glycol polymer (such as a polyethylene glycol polymer comprising poly vinyl acetate side chains) | from about 0 wt% to about 4 wt% |
| Polyester soil release polymer (such as Repel-O- Tex(R) and/or Texcare(R) polymers) | from about 0.1 wt% to about 2 wt% |
| Cellulosic polymer (such as carboxymethyl cellulose, methyl cellulose and combinations thereof) | from about 0.5wt% to about 2 wt% |
| Other polymer (such as amine polymers, dye transfer inhibitor polymers, hexamethylenediamine derivative polymers, and mixtures thereof) | from about 0 wt% to about 4 wt% |
| Zeolite builder and phosphate builder (such as zeolite 4A and/or sodium tripolyphosphate) | from about 0 wt% to about 4 wt% |

(continued)

| Ingredient | Amount |
|---|---|
| Other builder (such as sodium citrate and/or citric acid) | from about 0 wt% to about 3 wt% |
| Carbonate salt (such as sodium carbonate and/or sodium bicarbonate) | from about 15 t% to about 30 wt% |
| Silicate salt (such as sodium silicate) | from about 0 wt% to about 10 wt% |
| Filler (such as sodium sulphate and/or bio-fillers) | from about 10 wt% to about 40 wt% |
| Source of available oxygen (such as sodium percarbonate) | from about 10wt% to about 20 wt% |
| Bleach activator (such as tetraacetylethylene diamine (TAED) and/or nonanoyloxybenzenesulphonate (NOBS) | from about 2 wt% to about 8 wt% |
| Bleach catalyst (such as oxaziridinium-based bleach catalyst and/or transition metal bleach catalyst) | from about 0 wt% to about 0.1 wt% |
| Other bleach (such as reducing bleach and/or pre formed peracid) | from about 0 wt% to about 10 wt% |
| Chelant (such as ethylenediamine-N'N'-disuccinic acid (EDDS) and/or hydroxyethane diphosphonic acid (HEDP) | from about 0.2wt% to about 1wt% |
| Photobleach (such as zinc and/or aluminium sulphonated phthalocyanine) | from about 0 wt% to about 0.1 wt% |
| Hueing agent (such as direct violet 99, acid red 52, acid blue 80, direct violet 9, solvent violet 13 and any combination thereof) | from about 0 wt% to about 0.5 wt% |
| Brightener (such as brightener 15 and/or brightener 49) | from about 0.1wt% to about 0.4 wt% |
| Protease such as those mentioned under the heading "proteases" e.g. Savinase, Coronase, Ovozyme, Kannase, Liquanase, Polarzyme, Purafect, Properase, FN3, FN4 and any combination thereof, typically having an enzyme activity of from about 20 mg to about 100mg active enzyme/g) | from about 0.1wt% to about 1.5 wt% |
| Amylase (such as Termamyl(R), Termamyl Ultra(R), Natalase(R), Optisize HT Plus(R), Powerase (R), Stainzyme(R) and any combination thereof, typically having an enzyme activity of from about 10 mg to about 50 mg active enzyme/ g) | from about 0.05 wt% to about 0.2 wt% |
| Cellulase (such as Carezyme(R), Celluzyme(R) and/or Celluclean(R), typically having an enzyme activity of about from 10 to 50mg active enzyme/ g) | from about 0.05 wt% to 0.5 wt% |

(continued)

| Ingredient | Amount |
|---|---|
| Lipase (such as Lipex(R), Lipolex(R), Lipoclean(R) and any combination thereof, typically having an enzyme activity of from about 10 mg to about 50 mg active enzyme/ g) | from about 0.2 wt% to about 1 wt% |
| Other enzyme (such as xyloglucanase (e.g., Whitezyme(R)), cutinase, pectate lyase, mannanase, bleaching enzyme, typically having an enzyme activity of from about 10 mg to about 50 mg active enzyme/g) | from 0 wt% to 2 wt% |
| Fabric softener (such as montmorillonite clay and/or polydimethylsiloxane (PDMS)) | from 0 wt% to 15 wt% |
| Flocculant (such as polyethylene oxide) | from 0 wt% to 1 wt% |
| Suds suppressor (such as silicone and/or fatty acid) | from 0 wt% to 0.1wt% |
| Perfume (such as perfume microcapsule, spray-on perfume, starch encapsulated perfume accords, perfume loaded zeolite, and any combination thereof) | from 0.1 wt% to 1 wt% |
| Aesthetics (such as colored soap rings and/or colored speckles/noodles) | from 0 wt% to 1wt% |
| Miscellaneous | Balance |

All enzyme levels expressed as rug active enzyme protein per 100 g detergent composition. Surfactant ingredients can be obtained from BASF, Ludwigshafen, Germany (Lutensol(R)); Shell Chemicals, London, UK; Stepan, Northfield, Ill, USA; Huntsman, Huntsman, Salt Lake City, Utah, USA; Clariant, Sulzbach, Germany (Praepagen(R)).
Sodium tripolyphosphate can be obtained from Rhodia, Paris, France.
Zeolite can be obtained from Industrial Zeolite (UK) Ltd, Grays, Essex, UK.
Citric acid and sodium citrate can be obtained from Jungbunzlauer, Basel, Switzerland. NOBS is sodium nonanoyloxybenzenesulfonate, supplied by Eastman, Batesville, Ark., USA. TAED is tetraacetylethylenediamine, supplied under the Peractive(R) brand name by Clariant GmbH, Sulzbach, Germany.
Sodium carbonate and sodium bicarbonate can be obtained from Solvay, Brussels, Belgium. Polyacrylate, polyacrylate/maleate copolymers can be obtained from BASF, Ludwigshafen, Germany.
Repel-O-Tex(R) can be obtained from Rhodia, Paris, France.
Texcare(R) can be obtained from Clariant, Sulzbach, Germany. Sodium percarbonate and sodium carbonate can be obtained from Solvay, Houston, Tex., USA.
Na salt of Ethylenediamine-N,N'-disuccinic acid, (S,S) isomer (EDDS) was supplied by Octel, Ellesmere Port, UK.
Hydroxy ethane di phosphonate (HEDP) was supplied by Dow Chemical, Midland, Mich., USA. Enzymes Savinase(R), Savinase(R) Ultra, Stainzyme(R) Plus, Lipex(R), Lipolex(R), Lipoclean(R), Celluclean(R), Carezyme(R), Natalase(R), Stainzyme(R), Stainzyme(R) Plus, Termamyl(R), Termamyl(R) ultra, and Mannaway(R) can be obtained from Novozymes, Bagsvaerd, Denmark.
Enzymes Purafect(R), FN3 and FN4 can be obtained from DuPont International
Inc., Palo Alto, California, US. Direct violet 9 and 99 can be obtained from BASF DE, Ludwigshafen, Germany. Solvent violet 13 can be obtained from Ningbo Lixing Chemical Co., Ltd. Ningbo, Zhejiang, China. Brighteners can be obtained from Ciba Specialty Chemicals, Basel, Switzerland. All percentages and ratios are calculated by weight unless otherwise indicated. All percentages and ratios are calculated based on the total composition unless otherwise indicated.
It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

## WASH ASSAYS

### Launder-O-Meter (LOM) Model Wash System

The Launder-O-Meter (LOM) is a medium scale model wash system that can be applied to test up to 20 different wash conditions simultaneously. A LOM is basically a large temperature controlled water bath with 20 closed metal beakers rotating inside it. Each beaker constitutes one small washing machine and during an experiment, each will contain a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time consuming full scale experiments in front loader washing machines.

### Mini Launder-O-Meter (MiniLOM) Model Wash System

MiniLOM is a modified mini wash system of the Launder-O-Meter (LOM), which is a medium scale model wash system that can be applied to test up to 20 different wash conditions simultaneously. A LOM or is basically a large temperature controlled water bath with 20 closed metal beakers rotating inside it. Each beaker constitutes one small washing machine and during an experiment, each will contain a solution of a specific detergent/enzyme system to be tested along with the soiled and unsoiled fabrics it is tested on. Mechanical stress is achieved by the beakers being rotated in the water bath and by including metal balls in the beaker.

The LOM model wash system is mainly used in medium scale testing of detergents and enzymes at European wash conditions. In a LOM experiment, factors such as the ballast to soil ratio and the fabric to wash liquor ratio can be varied. Therefore, the LOM provides the link between small scale experiments, such as AMSA and mini-wash, and the more time consuming full scale experiments in front loader washing machines.

In miniLOM, washes are performed in 50 ml test tubes placed in Stuart rotator.

### Terg-O-Tometer (TOM) wash assay

The Terg-O-tometer (TOM) is a medium scale model wash system that can be applied to test 12 different wash conditions simultaneously. A TOM is basically a large temperature controlled water bath with up to 12 open metal beakers submerged into it. Each beaker constitutes one small top loader style washing machine and during an experiment, each of them will contain a solution of a specific detergent/enzyme system and the soiled and unsoiled fabrics its performance is tested on. Mechanical stress is achieved by a rotating stirring arm, which stirs the liquid within each beaker. Because the TOM beakers have no lid, it is possible to withdraw samples during a TOM experiment and assay for information on-line during wash.

The TOM model wash system is mainly used in medium scale testing of detergents and enzymes at US or LA/AP wash conditions. In a TOM experiment, factors such as the ballast to soil ratio and the fabric to wash liquor ratio can be varied. Therefore, the TOM provides the link between small scale experiments and the more time consuming full scale experiments in top loader washing machines.

## ENZYME ASSAYS

### Assay I: Testing of DNase activity

DNase activity was determined on DNase Test Agar with Methyl Green (BD, Franklin Lakes, NJ, USA), which was prepared according to the manual from supplier. Briefly, 21 g of agar was dissolved in 500 ml water and then autoclaved for 15 min at 121°C. Autoclaved agar was temperated to 48°C in water bath, and 20 ml of agar was poured into petri dishes with and allowed to solidify by incubation o/n at room temperature. On solidified agar plates, 5 μl of enzyme solutions are added, and DNase activity are observed as colorless zones around the spotted enzyme solutions.

## EXAMPLES

### EXAMPLE 1

**Evaluation of DNase anti-crease and softness properties**

In this study, heavy soiled Terry towelling towels and cotton T-shirts from Warwick Equest were used. Towels and T-shirts were divided into two equal parts. One part was washed with liquid Ariel Color & Style without DNase, whereas the second part was washed with liquid Ariel Color & Style with DNase.

Washes were done in Full Scale Wash (FSW) using Miele Softtronic W5841 washing machine (Program: Cottons; Additional program: Short; Temperature: 30°C; Centrifuge: 1600 rpm). Ariel Color & Style was dosed 5 g/L. Two DNases were tested. *Aspergillus oryzae* DNase (SEQ ID NO: 1) dosed at 2 ppm, and *Bacillus cibi* DNase (SEQ ID NO: 6) dosed at 0.2 ppm.

Towels and T-shirts were line-dried for 24 h at room temperature. Parts from same towel or T-shirt washed without and with DNase were collected and evaluated by a panel consisting of 1-4 panelists. Panelists were asked to select towel part being the softest and to select T-shirt part being the less creased. After evaluation, distribution was calculated.

Table 3. DNase treatment of heavy soiled Terry towelling towels and cotton T-shirts.

| Enzyme | Wash program | Towels (softness) | T-shirts (anti-crease) |
|---|---|---|---|
| *A. oryzae* DNase (2 ppm) | Short cotton wash, 30°C, Tap water | 79:21 | 80:20 |
| *B. cibi* DNase (0.2 ppm) | Short cotton wash, 30°C, Tap water | 69:31 | 75:25 |

The softness and anti-crease is indicated with X:Y values, wherein X specifies the % of the panelists that prefers real items washed with DNase, and Y specifies the % that prefers real item washed without DNase. The sum of the X and Y values is 100%.

### EXAMPLE 2

**DNase anti-crease effect on Polyester**

In this study, non-soiled W30A polyester fabric pieces in 22 cm x 15 cm were used. Three pieces of fabric was added to each 1L beaker, which went through a wash cycle with detergent (liquid Ariel Color & Style). One half of the beakers included DNase, and the other half of the beakers did not include DNase. Each result is the average of two beakers.

Washes were done in Terg-O-Tometer (150 rpm, 30°C, 20 min, 25 °dH water hardness). Detergent was dosed at 5 g/L. *Bacillus cibi* DNase (SEQ ID NO: 6) was dosed at 2 ppm. From each beaker, a fabric piece was subjected to each of the following drying methods:

1) Line-dried for 24 hours at room temperature;
2) Tumble-dried for 10 min in AEG/Electrolux Lavatherm TN95470 using Program: "Skabstørt, medium, 2.04h";
3) Table-dried for 24 hours at room temperature.

Pairs of fabric pieces from the same drying method, but with or without DNase treatment, were evaluated by a panel consisting of 11 panelists. Panelists were asked to select, for each drying method, which fabric piece of the pairs (+/- DNase treatment) was the less creased (wrinkled). After evaluation, distribution was calculated.

A similar set-up was used to wash non-soiled W30A polyester fabric pieces in a Terg-O-Tometer but including a rinse step for 10 minutes with softener Lenor (1.4% (v/v)) after wash.

After drying, the W30A fabric pieces were evaluated in pairs (+/- DNase treatment) by a sensory panel consisting of 3 panelists after the procedure mentioned above.

Table 4. DNase treatment of clean (non-soiled) W30A fabric.

| Drying | Rinse with water | Rinse with water and softener |
|---|---|---|
| Line dried | 100:0 | 100:0 |

(continued)

| Drying | Rinse with water | Rinse with water and softener |
|---|---|---|
| Tumble dried | 63:37 | 100:0 |
| Table dried | 91:9 | - |

The anti-crease effect is indicated with X:Y values, wherein X specifies the % of the panelists that prefers the fabric washed with DNase, and Y specifies the % that prefers the fabric washed without DNase. The sum of the X and Y values is 100%.

**EXAMPLE 3**

**DNase anti-crease effect after full scale wash and drying with tumble dryer**

In this study, four pieces of CN17 fabric, each sized at 40 cm x 20 cm and soiled with 8 grams of soil-mix including DNA (SBL-CFT from CFT), were added to each of two washing machines. One washing machine included DNase, and the other did not include DNase.

Washes were done using a Miele Softtronic W5841 full scale washing machine (Program: Cottons; Additional program: Short; Temperature: 30°C; Centrifuge: 1600 rpm; Ballast: 600-700 g clean 100% cotton T-shirts). Liquid Ariel Color & Style detergent was dosed at 5 g/L. *Bacillus cibi* DNase (SEQ ID NO: 6) was dosed at 2 ppm.

From each washing machine, two fabric pieces were subjected to each of the following drying methods:

1) Line-dried for 24 hours at room temperature;
2) Tumble-dried for 10 min.

Fabric pieces from the same drying method, but with or without DNase treatment, were evaluated in pairs by scanning the fabric pieces with a professional flatbed scanner (Epson Expression 10000XL). The scanned images were saved as single image JPG-files and imported into ImageJ 1.50i, which is a Java-based image processing software that can measure the area and pixel value statistics in pictures. From each scan a region of interest of the same size for each piece and containing >95% of each fabric piece was cropped and the cropped images were converted to 8-bit gray-scale. The ImageJ 1.50i default threshold method with red threshold color and dark background was chosen for noise reduction. A minimum threshold level of 210 and a maximum threshold value of 245 was chosen. Pixel values obtained with reduced noise were representing wrinkels. ImageJ software is free to download from https://imagej.nih.gov/ij/index.html.

Table 5. DNase treatment of soiled CN17 fabric.

| Drying | Textile creases measured by ImageJ scan processing | |
|---|---|---|
| | with DNase treatment | without DNase treatment |
| Line dried | 60% | 57% |
| Tumble dried | 45% | 23% |

The numbers in Table 5 specifies the % of the scanned textile area which is wrinkle-free.

**EXAMPLE 4**

**DNase anti-crease effect with DNA soil from soil ballast evaluated on tracer textile of two different cotton types**

In this study, 8 pieces of W80A unsoiled cotton fabric (CFT) in size 25 cm x 35 cm, 8 pieces of CN42 unsoiled cotton fabric (CFT) in size 25 cm x 35 cm, and 4 pieces of soil-ballast (SBL-CFT) in size 40 cm x 20 cm (equalizing 8 grams of soil), were added to each of two washing machines. One washing machine included DNase, and the other did not include DNase.

Washes were done using a Miele Softtronic W5841 European front loader full scale washing machine (Program: Cottons; Additional program: Short; Temperature: 30°C; Centrifuge: 1600 rpm; Ballast: 600-700 g 100% cotton T-shirts). Liquid Ariel Color & Style detergent was dosed at 5 g/L. *Aspergillus oryzae* DNase (SEQ ID NO: 1) was dosed at 2 ppm.

From each machine W80A and CN42 fabric pieces were line-dried for 24 hours at room temperature.

The W80A and CN42 fabric pieces were evaluated by scoring according to AATCC Test Method 124 using AATCC 3D Smoothness Appearance Replicas by a panel consisting of 3 panelists (the panel set-up was as close to AATCC TM124 as possible). Panelists were asked to compare each swatch with the AATCC smoothness standards ranking from SA value = 1 (very wrinkled standard) to SA value 5 = (completely smooth standard). After evaluation, average panel scores were calculated for each condition.

Table 6. Smoothness after DNase treatment according to AATCC TM124.

| Tracer textile | Average SA-value according to AATCC | |
|---|---|---|
| | with DNase treatment | without DNase treatment |
| W80A | 3.3 | 2.5 |
| CN42 | 3.3 | 2.8 |

## Claims

1. Use of a DNase to treat a fabric to provide improved softness and/or anti-crease properties.

2. Use according to claim 1, wherein the fabric is contacted with a liquid solution comprising a polypeptide having DNase activity.

3. Use according to any of claims 1-2, wherein the liquid solution is a wash liquor.

4. Use according to any of the preceding claims, wherein the fabric is a cellulosic fabric.

5. Use according to any of the preceding claims, wherein the DNase is a microbial DNase.

6. Use according to claim 5, wherein the DNase is a bacterial DNase.

7. Use according to claim 5, wherein the DNase is a fungal DNase.

8. Use according to claim 6, wherein the DNase is a polypeptide having an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8 and SEQ ID NO: 9 or a polypeptide having at least 60 % sequence identity hereto.

9. Use according to claim 7, wherein the DNase is a polypeptide having the amino acid sequence of SEQ ID NO: 1, or is a polypeptide having at least 60 % sequence identity hereto.

10. Use according to any of the preceding claims, wherein the DNase has at least 80% of the DNase activity of the DNase polypeptide of SEQ ID NO: 1 or SEQ ID NO: 4, when measured as described in Assay I.

11. Use according to any of the preceding claims, wherein the anti-crease effect ratio of test panelists preferring fabrics washed with DNase vs test panelists preferring fabrics washed without DNase is at least 60:40, preferably at least 70:30, preferably at least 80:20 or preferably at least 90:10, when measured as described in Example 1.

12. Use according to any of the preceding claims, wherein the improved softness effect ratio of test panelists preferring fabrics washed with DNase vs test panelists preferring fabrics washed without DNase is at least 60:40, preferably at least 70:30, preferably at least 80:20 or preferably at least 90:10, when measured as described in Example 1.

## Patentansprüche

1. Verwendung einer DNase, um ein Gewebe zu behandeln, um verbesserte Weichheit und/oder Knitterschutzeigenschaften bereitzustellen.

2. Verwendung nach Anspruch 1, wobei das Gewebe mit einer flüssigen Lösung in Kontakt gebracht wird, die ein

Polypeptid mit DNase-Aktivität umfasst.

3. Verwendung nach einem beliebigen der Ansprüche 1-2, wobei die flüssige Lösung eine Waschflotte ist.

4. Verwendung nach einem beliebigen der voranstehenden Ansprüche, wobei das Gewebe ein Cellulosegewebe ist.

5. Verwendung nach einem beliebigen der voranstehenden Ansprüche, wobei die DNase eine mikrobielle DNase ist.

6. Verwendung nach Anspruch 5, wobei die DNase eine bakterielle DNase ist.

7. Verwendung nach Anspruch 5, wobei die DNase eine pilzliche DNase ist.

8. Verwendung nach Anspruch 6, wobei die DNase ein Polypeptid mit einer Aminosäuresequenz ist, die aus der Gruppe bestehend aus SEQ ID NO: 2, SEQ ID NO: 3, SEQ ID NO: 4, SEQ ID NO: 5, SEQ ID NO: 6, SEQ ID NO: 7, SEQ ID NO: 8 und SEQ ID NO: 9 oder einem Polypeptid mit mindestens 60% Sequenzidentität dazu ausgewählt ist.

9. Verwendung nach Anspruch 7, wobei die DNase ein Polypeptid mit der Aminosäuresequenz von SEQ ID NO: 1 ist, oder ein Polypeptid mit mindestens 60% Sequenzidentität dazu ist.

10. Verwendung nach einem beliebigen der voranstehenden Ansprüche, wobei die DNase mindestens 80% der DNase-Aktivität des DNase-Polypeptids von SEQ ID NO: 1 oder SEQ ID NO: 4 aufweist, wenn sie wie in Assay I beschrieben gemessen wird.

11. Verwendung nach einem beliebigen der voranstehenden Ansprüche, wobei das Knitterschutzwirkungsverhältnis von Testgremiumsteilnehmern, die mit DNase gewaschene Gewebe bevorzugen, zu Testgremiumsteilnehmern, die ohne DNase gewaschene Gewebe bevorzugen, mindestens 60:40, vorzugsweise mindestens 70:30, vorzugsweise mindestens 80:20 oder vorzugsweise mindestens 90:10 beträgt, wenn es wie in Beispiel 1 beschrieben gemessen wird.

12. Verwendung nach einem beliebigen der voranstehenden Ansprüche, wobei das verbesserte Weichheitswirkungsverhältnis von Testgremiumsteilnehmern, die mit DNase gewaschene Gewebe bevorzugen, gegenüber Testgremiumsteilnehmern, die ohne DNase gewaschene Gewebe bevorzugen, mindestens 60:40, vorzugsweise mindestens 70:30, vorzugsweise mindestens 80:20 oder vorzugsweise mindestens 90:10 beträgt, wenn es wie in Beispiel 1 beschrieben gemessen wird.

## Revendications

1. Utilisation d'une DNase pour traiter une étoffe pour conférer une meilleure douceur et/ou des propriétés anti-plis.

2. Utilisation selon la revendication 1, dans laquelle l'étoffe est mise au contact d'une solution liquide comprenant un polypeptide ayant une activité de DNase.

3. Utilisation selon l'une quelconque des revendications 1 et 2, dans laquelle la solution liquide est une liqueur de lavage.

4. Utilisation selon l'une quelconque des revendications précédentes, dans laquelle l'étoffe est une étoffe cellulosique.

5. Utilisation selon l'une quelconque des revendications précédentes, dans laquelle la DNase est une DNase microbienne.

6. Utilisation selon la revendication 5, dans laquelle la DNase est une DNase bactérienne.

7. Utilisation selon la revendication 5, dans laquelle la DNase est une DNase fongique.

8. Utilisation selon la revendication 6, dans laquelle la DNase est un polypeptide ayant une séquence d'acides aminés choisie dans le groupe constitué par la SEQ ID NO : 2, la SEQ ID NO : 3, la SEQ ID NO : 4, la SEQ ID NO : 5, la SEQ ID NO : 6, la SEQ ID NO : 7, la SEQ ID NO : 8 et la SEQ ID NO : 9, ou un polypeptide ayant une identité de séquence d'au moins 60 % avec une telle séquence.

**9.** Utilisation selon la revendication 7, dans laquelle la DNase est un polypeptide ayant la séquence d'acides aminés de la SEQ ID NO : 1, ou est un polypeptide ayant une identité de séquence d'au moins 60 % avec une telle séquence.

**10.** Utilisation selon l'une quelconque des revendications précédentes, dans laquelle la DNase a au moins 80 % de l'activité de DNase du polypeptide DNase de la SEQ ID NO : 1 ou de la SEQ ID NO : 4, quand elle est mesurée comme décrit dans le Dosage I.

**11.** Utilisation selon l'une quelconque des revendications précédentes, dans laquelle le rapport d'effet anti-plis entre des panélistes de test préférant des étoffes lavées avec la DNase et des panélistes de test préférant des étoffes lavées sans la DNase est d'au moins 60/40, de préférence d'au moins 70/30, de préférence d'au moins 80/20 ou de préférence d'au moins 90/10, lors d'une mesure comme décrit dans l'Exemple 1.

**12.** Utilisation selon l'une quelconque des revendications précédentes, dans laquelle le rapport d'effet de meilleure douceur entre des panélistes de test préférant des étoffes lavées avec la DNase et des panélistes de test préférant des étoffes lavées sans la DNase est d'au moins 60/40, de préférence d'au moins 70/30, de préférence d'au moins 80/20 ou de préférence d'au moins 90/10, lors d'une mesure comme décrit dans l'Exemple 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009021989 A1
- WO 2011005963 A
- WO 2006097227 A
- WO 2011098579 A
- WO 2014087011 A
- WO 2015155350 A
- WO 201198579 A
- WO 09102854 A
- US 5977053 A
- WO 9817767 A
- EP 624154 A
- WO 2007087258 A
- WO 2007087244 A
- WO 2007087259 A
- EP 1867708 A
- WO 2007087242 A
- WO 2006130575 A
- WO 200503274 A
- WO 200503275 A
- WO 200503276 A
- EP 1876226 A
- WO 2007087257 A
- WO 2007087243 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- WO 99001544 A
- WO 2002099091 A
- WO 2001062903 A
- US 7262042 B
- WO 09021867 A
- WO 8906279 A
- WO 9318140 A
- WO 92175177 A
- WO 01016285 A
- WO 02026024 A
- WO 02016547 A
- WO 8906270 A
- WO 9425583 A
- WO 05040372 A
- WO 05052161 A
- WO 05052146 A
- WO 9523221 A
- WO 9221760 A
- EP 1921147 A
- EP 1921148 A
- WO 07044993 A
- WO 9219729 A
- WO 96034946 A
- WO 9820115 A
- WO 9820116 A
- WO 99011768 A
- WO 0144452 A
- WO 03006602 A
- WO 0403186 A
- WO 04041979 A
- WO 07006305 A
- WO 11036263 A
- WO 11036264 A
- WO 2016001449 A
- WO 2004067737 A
- US 5352604 A
- EP 258068 A
- EP 305216 A
- WO 9613580 A
- EP 218272 A
- EP 331376 A
- WO 9506720 A
- WO 9627002 A
- WO 9612012 A
- WO 10065455 A
- WO 10107560 A
- US 5389536 A
- WO 11084412 A
- WO 11084417 A
- WO 11084599 A
- WO 11150157 A
- WO 12137147 A
- EP 407225 A
- WO 9205249 A
- WO 9401541 A
- WO 9425578 A
- WO 9514783 A
- WO 9530744 A

- WO 9535381 A
- WO 9522615 A
- WO 9600292 A
- WO 9704079 A
- WO 9707202 A
- WO 0034450 A
- WO 0060063 A
- WO 0192502 A
- WO 0787508 A
- WO 09109500 A
- WO 10111143 A
- WO 0556782 A
- WO 0967279 A
- WO 10100028 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A
- US 4106991 A
- US 4661452 A
- GB 1483591 A
- EP 238216 A
- WO 2009087523 A
- WO 2007138054 A
- WO 2006108856 A
- WO 2006113314 A
- EP 1867808 A
- WO 2003040279 A
- WO 2008000333 A
- WO 9529218 A
- WO 2011011247 A
- WO 2009158388 A
- EP 2169040 A
- US 20090011970 A1
- WO 2013188331 A
- WO 2014032269 A


**Non-patent literature cited in the description**


- **NEEDLEMAN ; WUNSCH.** *J. Mol. Biol.,* 1970, vol. 48, 443-453
- EMBOSS: The European Molecular Biology Open Software Suite. **RICE et al.** Trends Genet. 2000, vol. 16, 276-277
- **RICE et al.** *EM-BOSS: The European Molecular Biology Open Software Suite,* 2000
- **SIEZEN et al.** *Protein Engng.,* 1991, vol. 4, 719-737
- **SIEZEN et al.** *Protein Science,* 1997, vol. 6, 501-523
- Surfactant science series. Powdered Detergents. Marcel Dekker, Inc, vol. 71